# EUROPEAN PATENT SPECIFICATION

(11) **EP 2 596 349 B1**
(45) Date of publication and mention of the grant of the patent: **13.12.2017**
(21) Application number: 11810457.9
(22) Date of filing: 22.07.2011
(51) Int. Cl.: G01N 33/48, G01N 33/53, C12Q 1/68

(54) **METHODS OF DETECTING CARDIOVASCULAR DISEASES OR CONDITIONS**
VERFAHREN ZUM NACHWEIS KARDIOVASKULÄRER ERKRANKUNGEN ODER LEIDEN
MÉTHODES DE DÉTECTION DE MALADIES OU DE PATHOLOGIES CARDIOVASCULAIRES

(30) Priority: 23.07.2010 US 367051 P
(43) Date of publication of application: 29.05.2013
(73) Proprietor: President and Fellows of Harvard College, Cambridge, MA 02138 (US)
(72) Inventor: KASSIS, Amin, I., Chestnut Hill Massachusetts 02467 (US)
(74) Representative: Regimbeau
(86) International application number: PCT/US2011/044996
(87) International publication number: WO 2012/012709

(56) References cited:
- WO-A1-2009/092068
- WO-A1-2009/092068
- US-A1- 2005 181 386
- US-A1- 2006 051 873
- US-A1- 2006 172 429
- US-A1- 2006 172 429
- US-A1- 2008 057 590
- ZAKYNTHINOS E ET AL: "Inflammatory biomarkers in coronary artery disease", JOURNAL OF CARDIOLOGY, NIHON SHINZOBYO GAKKAI, TOKYO, JP, vol. 53, no. 3, 1 June 2009 (2009-06-01), pages 317-333, XP026223375, ISSN: 0914-5087, DOI: 10.1016/J.JJCC.2008.12.007 [retrieved on 2009-01-29]

## Description

### Field of the Invention

Methods of using phagocytic cells alone or in combination with non-phagocytic cells in the diagnosis, prognosis, or monitoring of cardiovascular diseases or conditions, are herein provided. Methods of using phagocytic cells alone or in combination with non-phagocytic cells to identify markers of cardiovascular diseases or conditions, are also provided.

### Background

Early diagnosis of a disease often increases the likelihood of successful treatment or cure of such disease. Current diagnostic methods, however, depend largely on population-derived average values obtained from healthy individuals. Personalized diagnostic methods are needed that enable the diagnosis, especially the early diagnosis, of the presence of a disease or a condition in individuals who are not known to have the disease or who have recurrent disease. This is of particular importance in cardiovascular diseases or conditions, which are the leading cause of death in the United States.

Leukocytes begin as pluripotent hematopoietic stem cells in the bone marrow and develop along either the myeloid lineage (monocytes, macrophages, neutrophils, eosinophils, and basophils) or the lymphoid lineage (T and B lymphocytes and natural killer cells). The major function of the myeloid lineage cells (e.g., neutrophils and macrophages) is the phagocytosis of infectious organisms, live unwanted damaged cells, senescent and dead cells (apoptotic and necrotic), as well as the clearing of cellular debris. Phagocytes from healthy animals do not replicate and are diploid, i.e., have a DNA index of one. On average, each cell contains <10 ng DNA, <20 ng RNA, and <300 ng of protein.

One object is to provide diagnostic methods that can facilitate the detection of cardiovascular disease or condition-specific markers, e.g., nucleic acids, proteins, carbohydrates, and/or lipids and the like by using phagocytic cells alone, or in combination with non-phagocytic cells. Another object is to provide methods of identifying cardiovascular disease or condition-specific markers and further use such markers alone or together with any known markers to diagnose diseases or conditions.

### Summary of the Invention

The present invention is defined in claims 1 to 12.

We have invented new and useful methods for detecting/diagnosing cardiovascular diseases or conditions by using phagocytic cells alone or in combination with non-phagocytic cells. In some examples, phagocytic cells serve as surrogates for diseased cells and non-phagocytic cells serve as control cells. In other examples, two sub-populations of phagocytic cells are used, wherein the phagocytic cells that have a DNA content greater than 2n (the >2n phagocytic cells) serve as surrogates for diseased cells, while the phagocytic cells that have a DNA content of 2n (the =2n phagocytic cells) serve as control cells.

In one aspect, a method for diagnosing or aiding in the diagnosis of a cardiovascular disease or condition in a subject is provided, said method comprising: a) determining a first profile of one or more markers of the disease or condition from a population of phagocytic cells; b) determining a second profile of at least one of the one or more markers from a population of non-phagocytic cells; and c) identifying a difference between the first and second profiles of at least one or more of said markers, wherein the difference is indicative of the presence of said disease or condition in the subject.

In another aspect, a method for assessing the risk of developing a cardiovascular disease or condition in a subject is provided, said method comprising: a) determining a first profile of one or more markers of the disease or condition from a population of phagocytic cells; b) determining a second profile of at least one of the one or more markers from a population of non-phagocytic cells; and c) identifying a difference between the first and second profiles of at least one or more of said markers, wherein the difference is indicative of the risk of developing said disease or condition in the subject.

In yet another aspect, a method for prognosing or aiding in the prognosis of a cardiovascular disease or condition in a subject is provided, said method comprising: a) determining a first profile of one or more markers of the disease or condition from a population of phagocytic cells; b) determining a second profile of at least one of the one or more markers from a population of non-phagocytic cells; and c) identifying a difference between the first and second profiles of at least one or more of said markers, wherein the identified difference is indicative of the prognosis of said disease or condition in the subject.

In yet another aspect, a method for assessing the efficacy of a treatment for a cardiovascular disease or condition in a subject is provided, said method comprising: a) determining a first profile of one or more markers of the disease or condition from a population of phagocytic cells from the subject before the treatment; determining a second profile of at least one of the one or more markers from a population of non-phagocytic cells from the subject before the treatment; identifying a first difference between the first and second profiles of at least one or more of said markers; b) determining a third profile of the one or more markers from a population of phagocytic cells from the subject after the treatment; determining a fourth profile of at least one of the one or more markers from a population of non-phagocytic cells from the subject after the treatment; identifying a second difference between the third and fourth profiles of at least one or more of said markers; and c) identifying a difference between the first difference and the second difference, wherein the identified difference is indicative of the efficacy of the treatment for said disease or condition in the subject.

In yet another aspect, a method for monitoring the progression or regression of a cardiovascular disease or condition in a subject is provided, said method comprising: a) determining a first profile of one or more markers of the disease or condition from a population of phagocytic cells from the subject at a first time point; determining a second profile of at least one of the one or more markers from a population of non-phagocytic cells from the subject at the first time point; identifying a first difference between the first and second profiles of at least one or more of said markers; b) determining a third profile of the one or more markers from a population of phagocytic cells from the subject at a second time point; determining a fourth profile of at least one of the one or more markers from a population of non-phagocytic cells from the subject at the second time point; identifying a second difference between the third and fourth profiles of at least one or more of said markers; and c) identifying a difference between the first difference and the second difference, wherein the identified difference is indicative of the progression or regression of said disease or condition in the subject.

In yet another aspect, a method for identifying a compound capable of ameliorating or treating a cardiovascular disease or condition in a subject is provided, said method comprising: a) determining a first profile of one or more markers of the disease or condition from a population of phagocytic cells from the subject before administering the compound to the subject; determining a second profile of at least one of the one or more markers from a population of non-phagocytic cells from the subject before administering the compound to the subject; identifying a first difference between the first and second profiles of at least one or more of said markers; b) determining a third profile of the one or more markers from a population of phagocytic cells from the subject after the administration of the compound; determining a fourth profile of at least one of the one or more markers from a population of non-phagocytic cells from the subject after the administration of the compound; identifying a second difference between the third and fourth profiles of at least one or more of said markers; and c)identifying a difference between the first difference and the second difference, wherein the identified difference indicates that the compound is capable of ameliorating or treating said disease or condition in the subject.

In yet another aspect, a method for diagnosing or aiding in the diagnosis of a cardiovascular disease or condition in a subject is provided, said method comprising: a) determining a first profile of one or more markers of the disease or condition from a population of phagocytic cells having a DNA content more than 2n (>2n phagocytic cells); b) determining a second profile of at least one of the one or more markers from a population of phagocytic cells having a DNA content of 2n (=2n phagocytic cells); and c) identifying a difference between the first and second profiles of at least one or more of said markers, wherein the difference is indicative of the presence of said disease or condition in the subject.

In yet another aspect, a method for assessing the risk of developing a cardiovascular disease or condition in a subject is provided, said method comprising: a) determining a first profile of one or more markers of the disease or condition from a population of >2n phagocytic cells; b) determining a second profile of at least one of the one or more markers from a population of =2n phagocytic cells; and c) identifying a difference between the first and second profiles of at least one or more of said markers, wherein the difference is indicative of the risk of developing said disease or condition in the subject.

In yet another aspect, a method for prognosing or aiding in the prognosis of a cardiovascular disease or condition in a subject is provided, said method comprising: a) determining a first profile of one or more markers of the disease or condition from a population of >2n phagocytic cells; b) determining a second profile of at least one of the one or more markers from a population of =2n phagocytic cells; and c) identifying a difference between the first and second profiles of at least one or more of said markers, wherein the difference is indicative of the prognosis of said disease or condition in the subject.

In yet another aspect, a method for assessing the efficacy of a treatment for a cardiovascular disease or condition in a subject is provided, said method comprising: a) determining a first profile of one or more markers of the disease or condition from a population of>2n phagocytic cells from the subject before the treatment; determining a second profile of at least one of the one or more markers from a population of =2n phagocytic cells from the subject before the treatment; identifying a first difference between the first and second profiles of at least one or more of said markers; b) determining a third profile of the one or more markers from a population of >2n phagocytic cells from the subject after the treatment; determining a fourth profile of at least one of the one or more markers from a population of =2n phagocytic cells from the subject after the treatment; identifying a second difference between the third and fourth profiles of at least one or more of said markers; and c) identifying a difference between the first difference and the second difference, wherein the identified difference is indicative of the efficacy of the treatment for said disease or condition in the subject.

In yet another aspect, a method for monitoring the progression or regression of a cardiovascular disease or condition in a subject is provided, said method comprising: a) determining a first profile of one or more markers of the disease or condition from a population of>2n phagocytic cells from the subject at a first time point; determining a second profile of at least one of the one or more markers from a population of =2n phagocytic cells from the subject at the first time point; identifying a first difference between the first and second profiles of at least one or more of said markers; b) determining a third profile of the one or more markers from a population of>2n phagocytic cells from the subject at a second time point; determining a fourth profile of at least one of the one or more markers from a population of =2n phagocytic cells from the subject at the second time point; identifying a second difference between the third and fourth profiles of at least one or more of said markers; and c) identifying a difference between the first difference and the second difference, wherein the identified difference is indicative of the progression or regression of said disease or condition in the subject.

In yet another aspect, a method for identifying a compound capable of ameliorating or treating a cardiovascular disease or condition in a subject is provided, said method comprising: a) determining a first profile of one or more markers of the disease or condition from a population of>2n phagocytic cells from the subject before administering the compound to the subject; determining a second profile of at least one of the one or more markers from a population of =2n phagocytic cells from the subject before administering the compound to the subject; identifying a first difference between the first and second profiles of at least one or more of said markers; b) determining a third profile of the one or more markers from a population of>2n phagocytic cells from the subject after the administration of the compound; determining a fourth profile of at least one of the one or more markers from a population of =2n phagocytic cells from the subject after the administration of the compound; identifying a second difference between the third and fourth profiles of at least one or more of said markers; c) identifying a difference between the first difference and the second difference, wherein the identified difference indicates that the compound is capable of ameliorating or treating said disease or condition in the subject.

In yet another aspect, a method for identifying one or more markers for a cardiovascular disease or condition is provided, said method comprising: a) determining a first profile of analytes from phagocytic cells from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from phagocytic cells from a control subject not having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. Optionally, this method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, a method for identifying one or more markers of a cardiovascular disease or condition is provided, said method comprising: a) determining a first profile of analytes from phagocytic cells from a subject having said disease or condition; determining a second profile of analytes from phagocytic cells from a control subject not having said disease or condition; identifying a first set of differences between the first and second profiles, wherein the first set of differences is specific to the first profile relative to the second profile; b) determining a third profile of analytes from non-phagocytic cells from the subject having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, a method for identifying one or more markers of a cardiovascular disease or condition is provided, said method comprising: a) determining a first profile of analytes from phagocytic cells from a subject having said disease or condition; obtaining a second profile of analytes from phagocytic cells from a control subject not having said disease or condition by data mining; identifying a first set of differences between the first and second profiles, wherein the first set of differences is specific to the first profile relative to the second profile; b) determining a third profile of analytes from non-phagocytic cells from the subject having said disease or condition; obtaining a fourth profile of analytes from non-phagocytic cells from a control subject not having said disease or condition by data mining; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition by data mining; obtaining a sixth profile of analytes from cells or tissues not affected by said disease or condition by data mining; identifying a third set of differences between the fifth and sixth profiles, wherein the third set of differences is specific to the fifth profile relative to the sixth profile; and e) identifying at least one of the one or more markers of c) present in the third set of differences.

In yet another aspect, a method for identifying one or more markers of a cardiovascular disease or condition is provided, said method comprising: a) determining a first profile of analytes from phagocytic cells from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from cells or tissues affected by said disease or condition from the subject having said disease or condition; determining a fourth profile of analytes from cells or tissues not affected by said disease or condition from the subject having said disease or condition; identifying a second set of differences between the third and fourth profiles, wherein the second set of differences is specific to the third profile relative to the fourth profile; c) identifying one or more analytes present in both the first set of differences and the second set of differences, the identified analytes being markers of said disease or condition. And optionally, the method further comprises d) determining a fifth profile of analytes from phagocytic cells from a control subject not having said disease or condition; identifying a third set of differences between the first and fifth profiles, wherein the third set of differences is specific to the first profile relative to the fifth profile; e) identifying at least one of the one or more markers of c) present in the third set of differences.

In yet another aspect, a method for identifying one or more markers of a cardiovascular disease or condition is provided, said method comprising: a) determining a first profile of analytes from >2n phagocytic cells from a subject having said disease or condition; determining a second profile of analytes from =2n phagocytic cells from the subject having said disease or condition;
identifying a first set of differences between the first and second profiles, wherein the first set of differences is specific to the first profile relative to the second profile; b) determining a third profile of analytes from >2n phagocytic cells from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises: d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In some embodiments, the markers or the analytes are nucleic acids (e.g., nucleotides, oligonucleotides, DNAs, RNAs, or DNA-RNA hybrids), proteins (e.g., , amino acids, peptides, enzymes, antigens, antibodies, cytokines, lipoproteins, glycoproteins, or hormones), lipids (e.g., fatty acids, phosphatides, cholesterol), carbohydrates (e.g., monosaccharides, disaccharides, polysaccharides), metabolites (e.g., vitamins, primary metabolites, secondary metabolites), or combinations thereof.

In some embodiments, the profile is a nucleic acid profile (e.g., genotypic profile, a single nucleotide polymorphism profile, a gene mutation profile, a gene copy number profile, a DNA methylation profile, a DNA acetylation profile, a chromosome dosage profile, a gene expression profile), a protein profile (e.g., protein expression, protein activation), a lipid profile, a carbohydrate profile, a metabolite profile, or a combination thereof. In some embodiments, the profile is determined by a qualitative assay, a quantitative assay, or a combination thereof.

In some particular examples, at least one of the one or more markers is up-regulated or activated in the phagocytic cells compared to the non-phagocytic cells. In some particular examples, at least one of the one or more markers is down-regulated or inhibited in the phagocytic cells compared to the non-phagocytic cells. In some particular examples, at least one of the one or more markers is up-regulated or activated in the >2n phagocytic cells compared to the =2n phagocytic cells. In some particular examples, at least one of the one or more markers is down-regulated or inhibited in the >2n phagocytic cells compared to the =2n phagocytic cells.

In some particular examples, the first profile, the second profile, the third profile, the fourth profile, the fifth profile, or the sixth profile comprises the absence of at least one of the one or more markers.

In some particular examples, the difference is at least 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference.

In some particular examples, the methods of this invention also comprise lysing the phagocytic cells (e.g., >2n phagocytic cells, or =2n phagocytic cells), and the non-phagocytic cells; and also extracting the cellular contents from those cells. In some embodiments, the cellular contents of the phagocytic cells comprise viable diseased cells, dead diseased cells, apoptotic diseased cells, circulating tumor cells, infectious agents, fetal cells, trophoblasts, or fragments thereof. In some particular examples, the cellular contents of the >2n phagocytic cells comprise viable diseased cells, dead diseased cells, apoptotic diseased cells, circulating tumor cells, infectious agents, fetal cells, trophoblasts, or fragments thereof.

In some particular examples, at least one of the one or more markers of the disease or condition is present in the cellular contents of the phagocytic cells. In some particular examples, the one or more markers of said disease or condition are not present in the cellular contents of the non phagocytic cells. In some particular examples, the phagocytic cells express at least one of the one or more markers of said disease or condition.

In some particular examples, at least one of the one or more markers of the disease or condition is present in the cellular contents of the >2n phagocytic cells. In some particular examples, the one or more markers of said disease or condition are not present in the cellular contents of the =2n phagocytic cells. In some particular examples, the phagocytic cells express at least one of the one or more markers of said disease or condition. In some particular examples, the >2n phagocytic cells express at least one of the one or more markers of said disease or condition.

In some examples, the methods of this invention also comprise comparing the identified difference of c) to a repository of one or more known markers of said disease or condition (e.g., data obtained by data mining).

In some embodiments, the phagocytic cells are professional phagocytic cells (e.g., neutrophils, macrophages, monocytes, dendritic cells, foam cells, mast cells, eosinophils), non-professional phagocytic cells (e.g., epithelial cells, endothelial cells, fibroblasts, mesenchymal cells), or mixtures thereof. In some embodiments, the non-phagocytic cells are T cells, B cells, null cells, basophils, or mixtures thereof.

In some particular examples, the phagocytic cells (e.g., >2n phagocytic cells, =2n phagocytic cells) and the non-phagocytic cells are isolated from a bodily fluid sample (e.g., blood, urine), tissues, or cells (e.g., white blood cells, fetal cells) of the subject.

In some examples, a standard/known cell separation/isolation/purification technique, such as antibody, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platforms, or a combination thereof, is used to isolate phagocytic cells (e.g., >2n phagocytic cells and =2n phagocytic cells) and non-phagocytic cells from bodily fluids, tissues or cells, or to separate phagocytic cells from non-phagocytic cells, or to separate >2n phagocytic cells from =2n phagocytic cells. In some examples, the phagocytic cells (e.g., >2n phagocytic cells) can also be isolated by using a product secreted by the phagocytic cells, or by using a cell surface target (e.g., a receptor protein, a marker of said disease or condition) on the surface of the phagocytic cells. In some examples, the target is expressed by the phagocytic cells. In other examples, the target is not expressed by the phagocytic cells. In some examples, the phagocytic cells (e.g., >2n phagocytic cells and =2n phagocytic cells) and the non-phagocytic cells are isolated using a ligand that binds to a molecular receptor expressed on the plasma membranes of white blood cells.

Also provided herein are markers that can be used in the instant methods and that can be identified by the instant methods.

### Brief Description of the Drawings

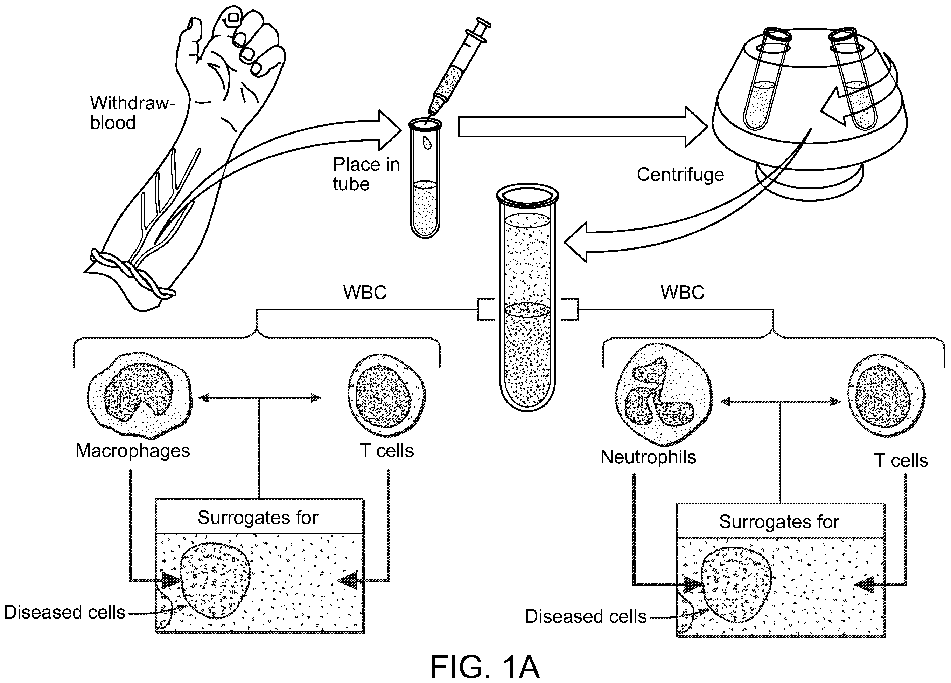

**FIG. 1A** schematically depicts one example of a method of the invention for diagnosing or aiding in the diagnosis of a cardiovascular disease or condition. In this example, phagocytic cells and non-phagocytic cells are separated from white blood cells of a subject. The phagocytic cells serve as surrogates for diseased cells, while the non-phagocytic cells serve as control cells.

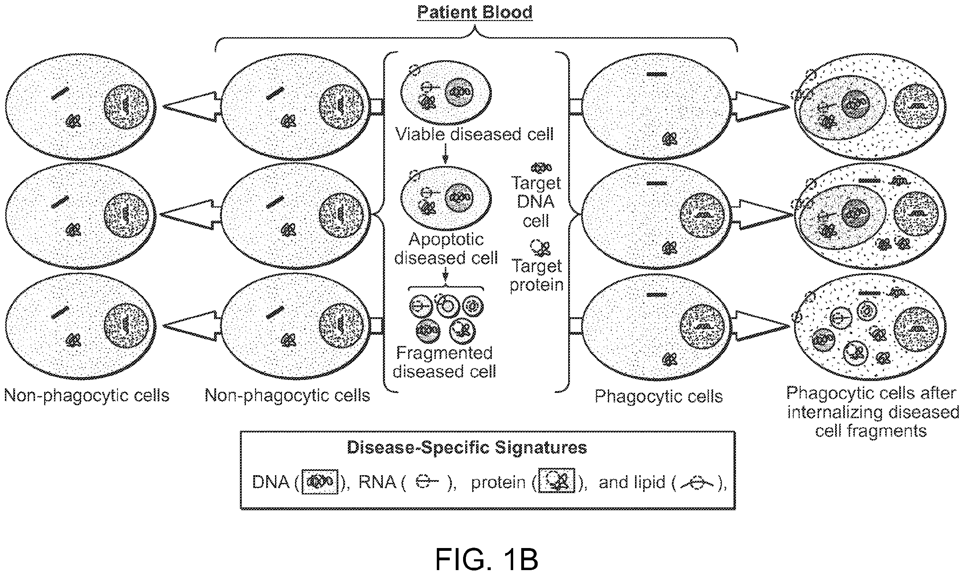

**FIG. 1B** schematically depicts one proposed pathway leading to acquisition of a cardiovascular disease-specific DNA, RNA, protein and/or lipid markers by phagocytic cells. Blood phagocytes engulf viable circulating diseased cells, apoptotic diseased cells, and/or fragmented diseased cells. Accordingly, the disease-specific markers (e.g., DNAs, RNAs, proteins, or lipids) that are contained within these diseased cells/fragments are also internalized by phagocytic cells. By contrast, non-phagocytic cells do not internalize these diseased cells/fragments, and, therefore, do not contain and/or express the disease-specific markers.

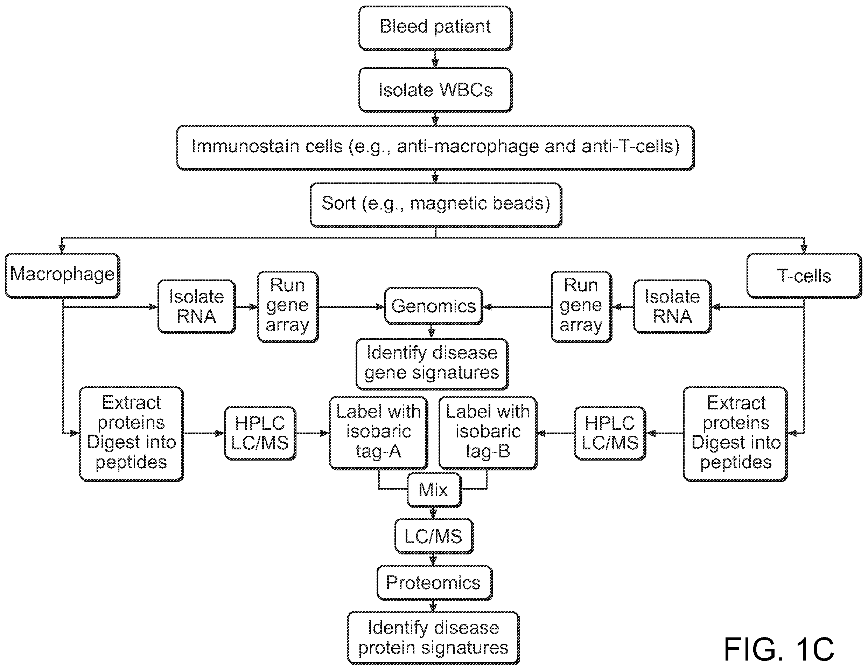

**FIG. 1C** schematically depicts a general flowchart of one example of a method of the invention.

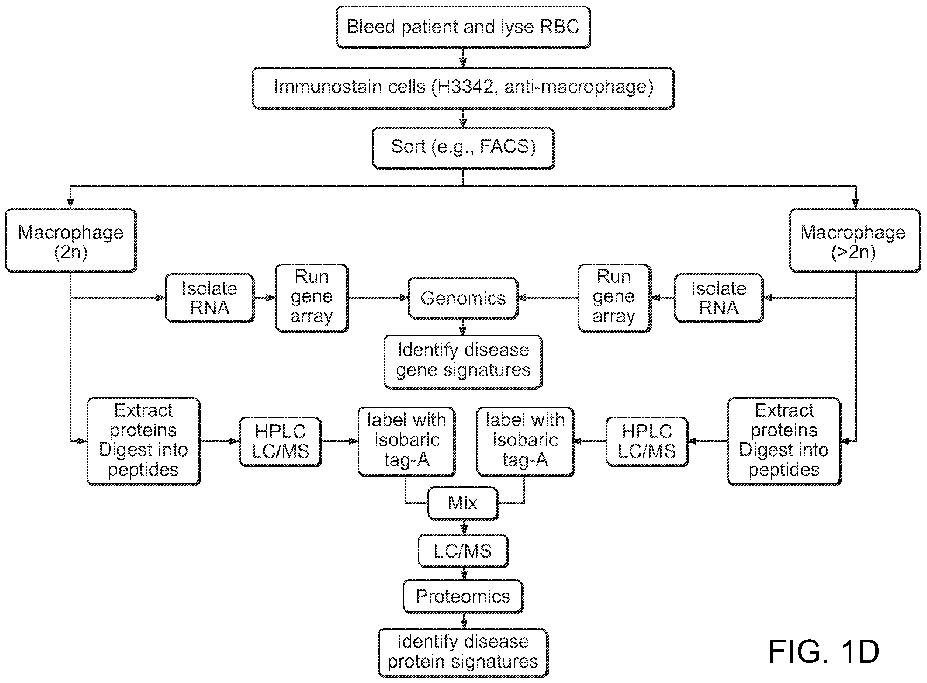

**FIG. 1D** schematically depicts a general flowchart of one example of a method of the invention.

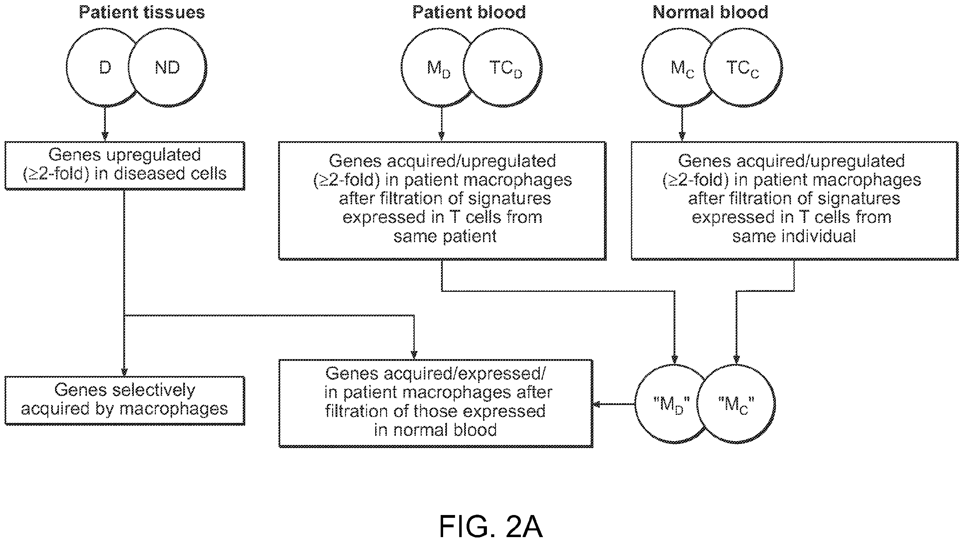

**FIG. 2A** schematically depicts one particular example of a method of this invention for identifying one or more markers of a cardiovascular disease or condition. D represents diseased cells/tissues from a patient having a cardiovascular disease or condition; and ND represents not-diseased cells/tissues from the patient; M_{D} represents macrophages taken from the patient; TC_{D} represents T cells taken from the patient(s); M_{C} represents macrophages taken from a control subject not having the disease or condition; TC_{C} represents T cells taken from the control subject.

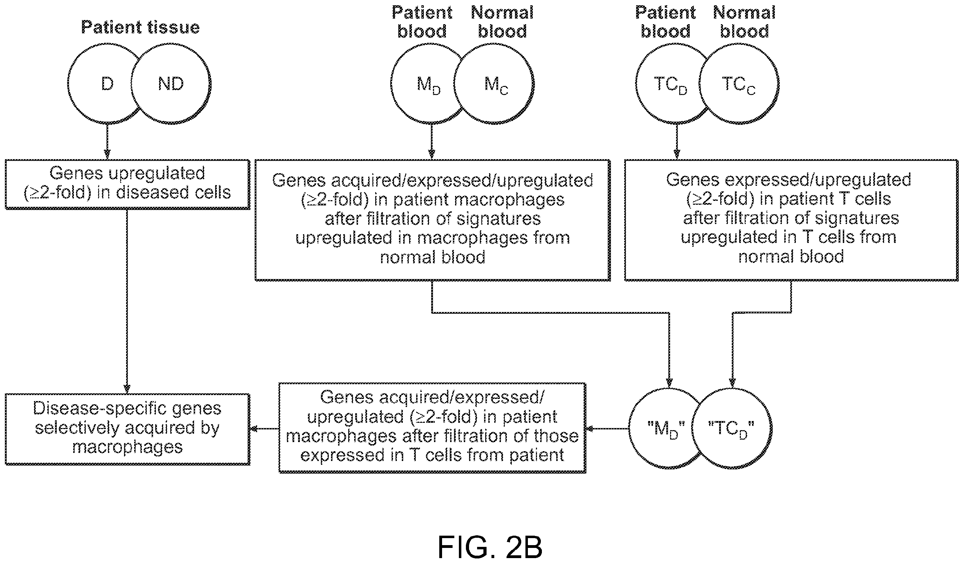

**FIG. 2B** schematically depicts one particular example of a method of this invention for identifying one or more markers of a cardiovascular disease or condition. D represents diseased cells/tissues from a patient having a cardiovascular disease or condition; and ND represents not-diseased cells/tissues from the patient; M_{D} represents macrophages taken from the patient; TC_{D} represents T cells taken from the patient; M_{C} represents macrophages taken from a control subject not having the disease or condition; TC_{C} represents T cells taken from the control subject.

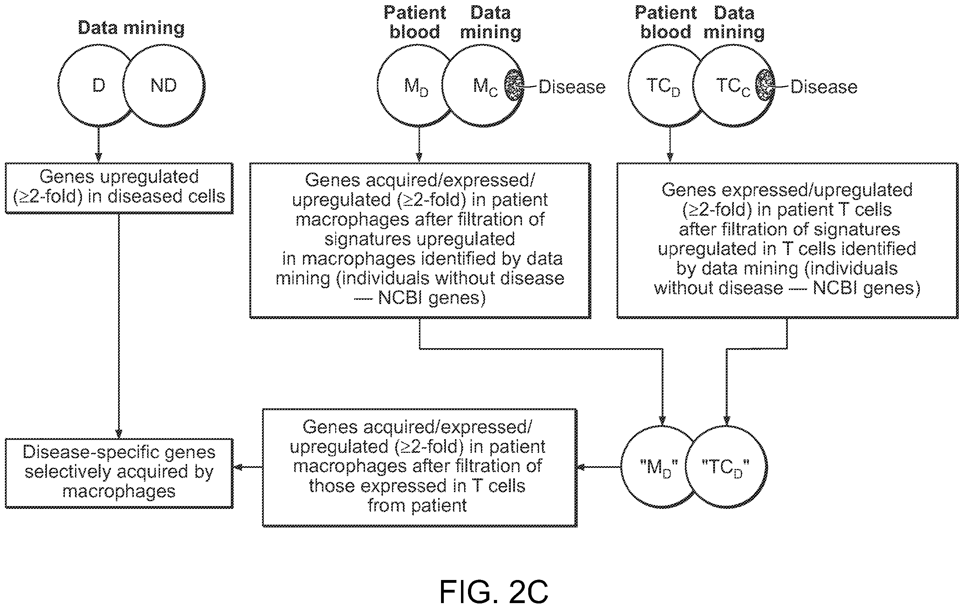

**FIG. 2C** schematically depicts one particular example of a method of this invention for identifying one or more markers of a cardiovascular disease or condition. D represents information obtained by data mining about diseased cells/tissues from patients having a cardiovascular disease or condition; and ND represents information obtained by data mining about not-diseased cells/tissues from patients having the same disease or condition; M_{D} represents macrophages taken from a patient having the disease or condition; TC_{D} represents T cells taken from the patient; M_{C} represents information obtained by data mining about macrophages from control subjects not having the disease or condition; TC_{C} represents information obtained by data mining about T cells obtained from control subjects not having the disease or condition.

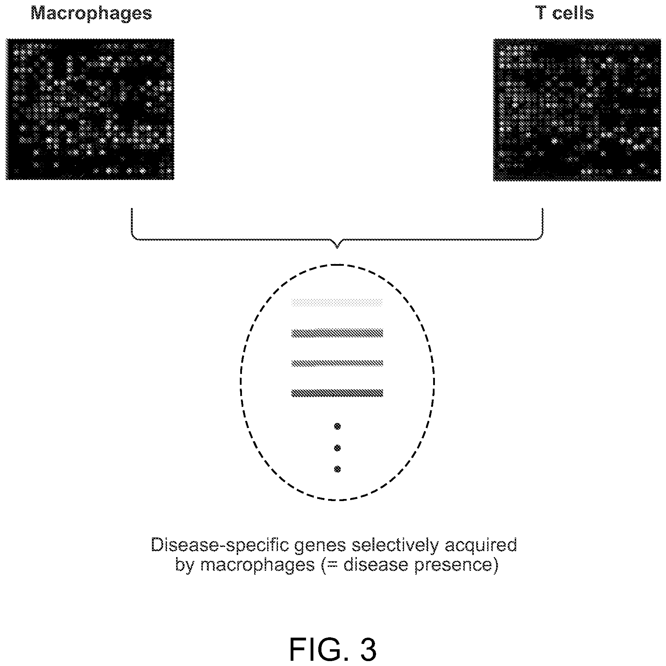

**FIG. 3** depicts a schematic of gene expression profile data that could be compared to identify cardiovascular disease-specific genes selectively acquired/expressed by macrophages.

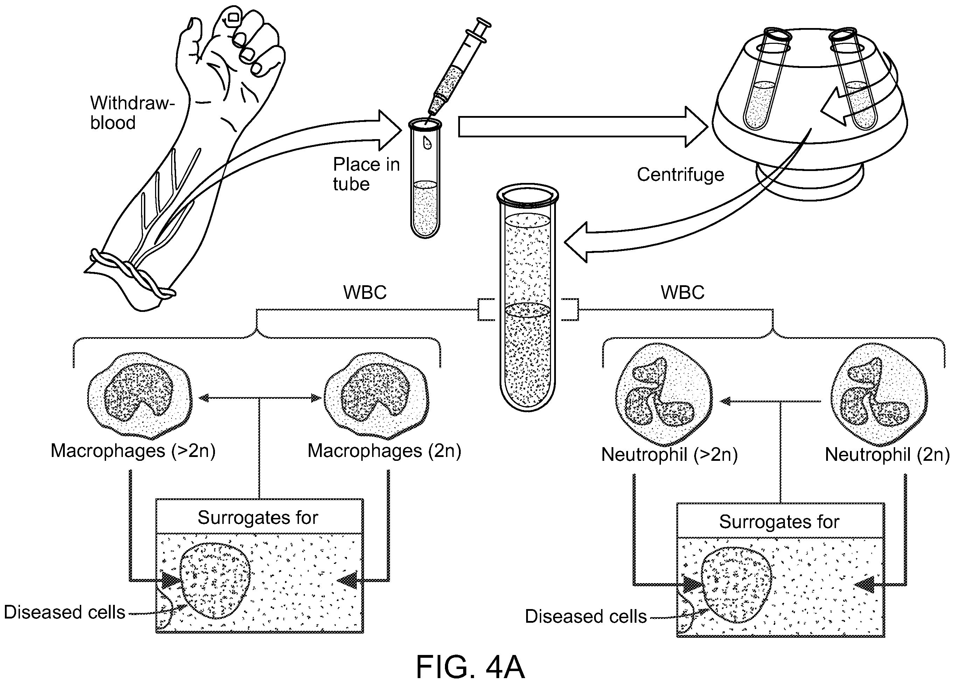

**FIG. 4A** schematically depicts one example of a method of this invention for diagnosing or aiding in the diagnosis of a cardiovascular disease or condition. In this example, a blood sample is withdrawn from an individual to be diagnosed. After a centrifugation step, white blood cells are isolated from the blood sample and further separated into two populations of phagocytic cells: phagocytic cells (e.g., macrophages or neutrophils) having a DNA content more than 2n (>2n phagocytic cells) and phagocytic cells (e.g., macrophages or neutrophils) having a DNA content of 2n (=2n phagocytic cells). The >2n phagocytic cells serve as surrogates for diseased cells and the 2n phagocytic cells serve as control cells.

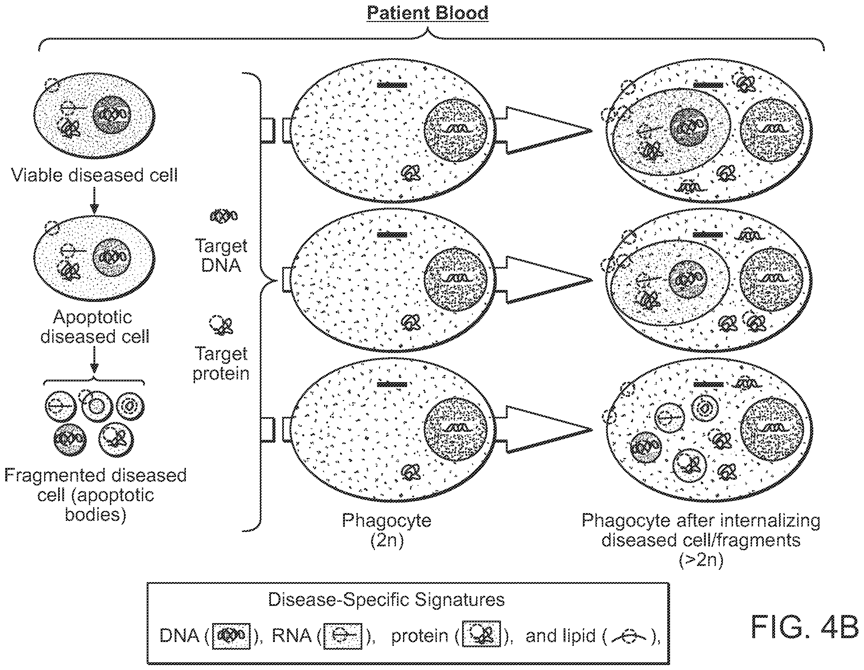

**FIG. 4B** schematically depicts one proposed pathway leading to acquisition of cardiovascular disease or condition-specific markers (e.g., DNA, RNA, protein and lipid markers) by phagocytic cells. Blood phagocytes engulf viable circulating diseased cells, apoptotic diseased cells, and/or fragmented diseased cells. Accordingly, the cardiovascular disease or condition-specific markers (e.g., DNAs, RNAs, proteins, or lipids) that are contained within these diseased cells/fragments are also internalized by phagocytic cells, which then become >2n phagocytic cells containing and/or expressing these specific markers. By contrast, phagocytic cells that do not internalize these diseased cells/fragments, and thus, do not contain or express these markers, and remain DNA content of 2n.

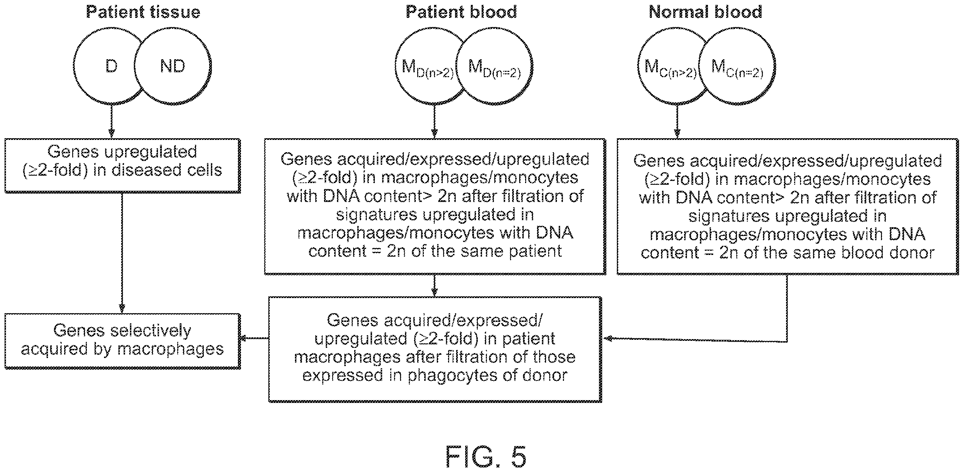

**FIG. 5** schematically depicts one particular example of a method of this invention for identifying one or more markers of a cardiovascular disease or condition. D represents diseased tissues/cells from a patient having a cardiovascular disease or condition; and ND represents not-diseased tissues/cells from the patient; M_{D(N>2)} represents macrophages having a DNA content of >2n taken from a patient with the disease or condition; M_{D(N=2)} represents macrophages having a DNA content of =2n taken from the patient; M_{C(N>2)} represents macrophages having a DNA content of>2n taken from a control subject not having the disease or condition; M_{C(N=2)} represents macrophages having a DNA content of>2n taken from the control subject.

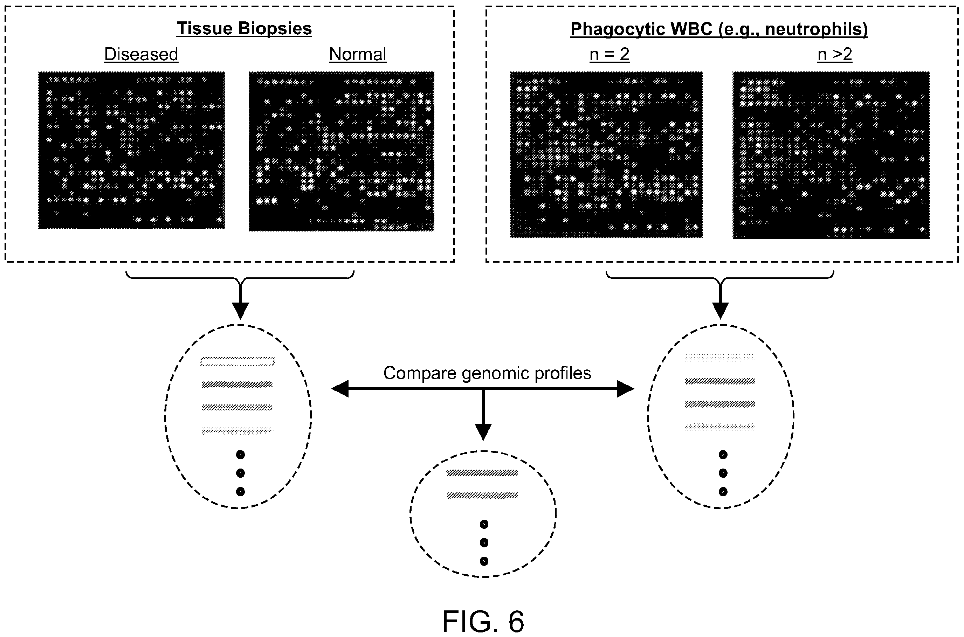

**FIG. 6** schematically depicts one example of a method of this invention for identifying cardiovascular disease or condition-specific markers selectively acquired/expressed by >2n phagocytic cells of a patient.

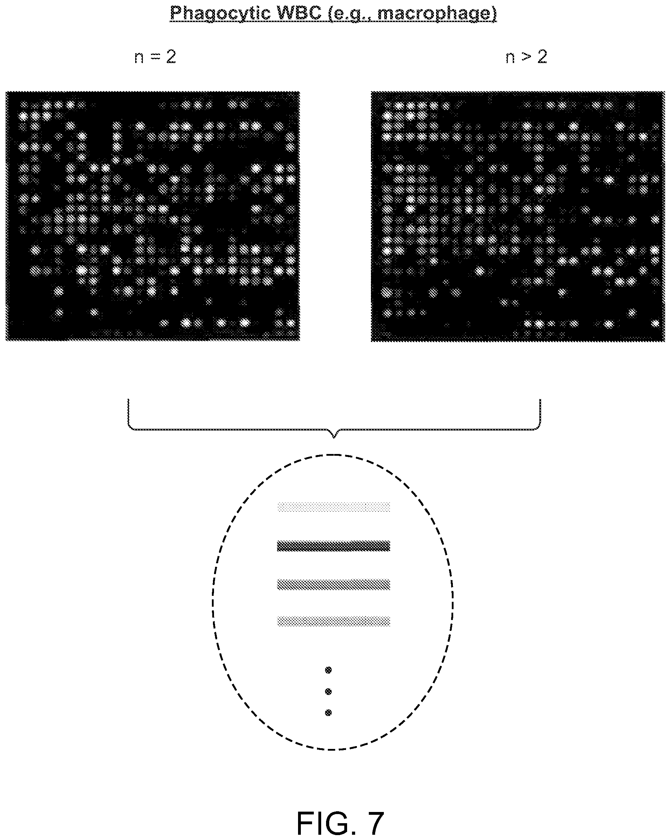

**FIG. 7** schematically depicts one example of a method of this invention for diagnosing/detecting a cardiovascular disease or condition by comparing expression profiles obtained from arrays.

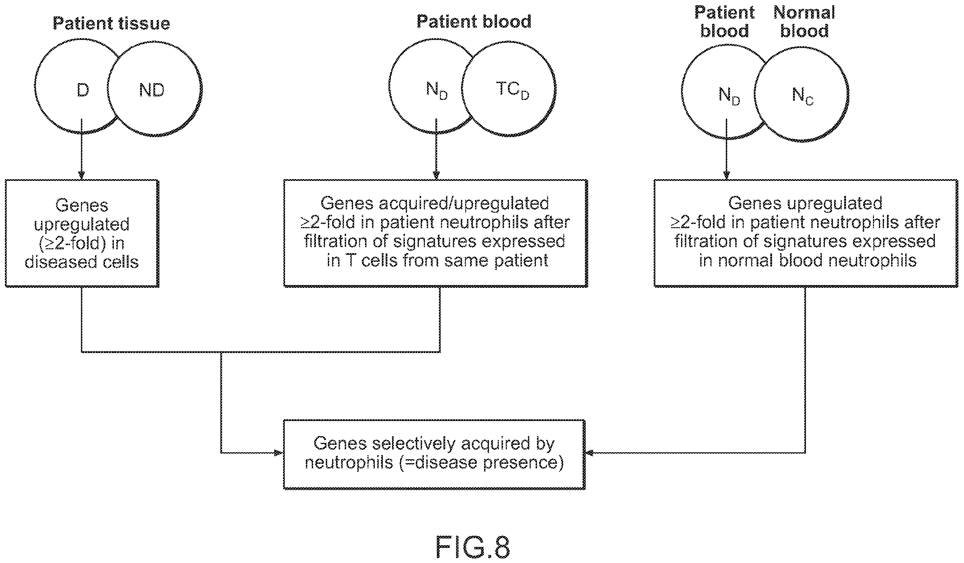

**FIG. 8** schematically depicts one particular example of a method of this invention for identifying one or more markers of a cardiovascular disease or condition. D represents diseased cells/tissues from a patient having a cardiovascular disease or condition; and ND represents not-diseased cells/tissues from the patient; N_{D} represents neutrophils taken from the patient; TC_{D} represents T cells taken from the patient; N_{C} represents neutrophils obtained from a control subject not having the disease or condition.

### Detailed Description of the Invention

Unless otherwise defined herein, scientific and technical terms used in this application shall have the meanings that are commonly understood by those of ordinary skill in the art. Generally, nomenclature used in connection with, and techniques of, cell and tissue culture, molecular biology, cell and cancer biology, neurobiology, neurochemistry, virology, immunology, microbiology, pharmacology, genetics and protein and nucleic acid chemistry, described herein, are those well-known and commonly used in the art.

Throughout this specification, the word "comprise" or variations such as "comprises" or "comprising" will be understood to imply the inclusion of a stated integer (or components) or group of integers (or components), but not the exclusion of any other integer (or components) or group of integers (or components).

The singular forms "a," "an," and "the" include the plurals unless the context clearly dictates otherwise.

The term "including" is used to mean "including but not limited to". "Including" and "including but not limited to" are used interchangeably.

A "patient", "subject", or "individual" are used interchangeably and refer to either a human or a non-human animal. These terms include mammals, such as humans, primates, livestock animals (e.g., bovines, porcines), companion animals (e.g., canines, felines) and rodents (e.g., mice and rats).

As used herein, a control subject refers to any individual that has not been diagnosed as having the disease or condition being assayed. The terms "normal control", "healthy control", and "not-diseased cells" likewise mean a sample (e.g., cells, serum, tissue) taken from a source (e.g., subject, control subject, cell line) that does not have the condition or disease being assayed and therefore may be used to determine the baseline for the condition or disorder being measured. It is also understood that the control subject, normal control, and healthy control, include data obtained and used as a standard, i.e. it can be used over and over again for multiple different subjects. In other words, for example, when comparing a subject sample to a control sample, the data from the control sample could have been obtained in a different set of experiments, for example, it could be an average obtained from a number of healthy subjects and not actually obtained at the time the data for the subject was obtained.

The term "diagnosis" as used herein refers to methods by which the skilled artisan can estimate and/or determine whether or not a patient is suffering from a given disease or condition. The skilled artisan often makes a diagnosis on the basis of one or more diagnostic indicators, e.g., a marker, the presence, absence, amount, or change in amount of which is indicative of the presence, severity, or absence of the condition.

The term "prognosis" as used herein refers to is used herein to refer to the likelihood of a cardiovascular disease or condition progression, including recurrence of a disease or condition.

### Description of the Instant Methods

Methods for diagnosing or aiding in the diagnosis of a cardiovascular disease or condition by comparing profiles (e.g., gene/protein/lipid/carbohydrate expression profiles, genotypes, gene copy number, gene dosage, DNA methylation, etc.) of disease or condition-associated markers (e.g., nucleic acids, proteins, lipids, carbohydrates, metabolites) between phagocytic cells having different DNA contents (>2n vs. =2n) taken from the same individual, or between phagocytic cells and non-phagocytic cells taken from the same individual are provided herein.

Methods for assessing the risk of developing a cardiovascular disease or condition, prognosing said disease, monitoring said disease progression or regression, assessing the efficacy of a treatment, or identifying a compound capable of ameliorating or treating said disease or condition, are also provided herein.

Such a subject-specific profile comparison eliminates the dependence on a population-derived average profile for a particular disease or condition, which may introduce error into the detection or diagnosis of a particular disease or condition in the subject. These methods allow detection, diagnosis, and treatment to be personalized to the individual.

The present methods (i) have high specificity, sensitivity, and accuracy and are capable of detecting disease or condition-specific markers present within a bodily fluid sample, cells or tissues; and (ii) eliminate the "inequality of baseline" that is known to occur among individuals due to intrinsic (e.g., age, gender, ethnic background, health status and the like) and temporal variations in marker expression. Accordingly, in certain aspects, the invention provides non-invasive assays for the early detection of a disease or condition, i.e., before the disease can be diagnosed by conventional diagnostic techniques, e.g., imaging techniques, and, therefore, provide a foundation for improved decision-making relative to the needs and strategies for intervention, prevention, and treatment of individuals with such disease or condition.

The present methods can be used together with any known diagnostic methods, such as physical inspection, visual inspection, biopsy, scanning, histology, radiology, imaging, ultrasound, use of a commercial kit, genetic testing, immunological testing, analysis of bodily fluids, or monitoring neural activity.

Phagocytic cells that can be used in the methods include all types of cells that are capable of ingesting various types of substances (e.g., apoptotic cells, infectious agents, dead cells, viable cells, cell-free DNAs, cell-free RNAs, cell-free proteins). In some embodiments, the phagocytic cells are professional phagocytic cells, such as neutrophils, macrophages, monocytes, dendritic cells, foam cells, mast cells, or eosinophils. In some embodiments, the phagocytic cells are non-professional phagocytic cells, such as epithelial cells, endothelial cells, fibroblasts, or mesenchymal cells. In other embodiments, the phagocytic cells can be a mixture of different types of phagocytic cells. Non-phagocytic cells that can be used in this invention include, but are not limited to, T cells, B cells, null cells, basophils, or mixtures thereof.

As used herein, "the >2n phagocytic cells" refer to phagocytic cells that have a DNA content of greater than 2n, while "the =2n phagocytic cells" refer to phagocytic cells that have a DNA content of 2n. Some phagocytic cells engulf live/dying/dead diseased cells (and sub-cellular fragments thereof) and/or cell-free disease-specific nucleic acids, proteins, carbohydrates and/or lipids present in bodily fluids. Such phagocytosis leads to the internalization of these disease markers into the phagocytic cell and, therefore, the DNA content of these phagocytic cells will become greater than 2n. By contrast, some phagocytic cells have not engulfed living/dying/dead diseased cells or fragments and/or cell-free disease-specific nucleic acids, proteins, lipids, and/or carbohydrates present in bodily fluids. The DNA contents of this group of phagocytic cells remain 2n. In some embodiments, the disease-specific markers (e.g., DNA with disease-specific mutations) can be expressed by the >2n phagocytic cells. For example, the mutated DNA of diseased cells is integrated into the normal DNA of the >2n phagocytic cells. The subsequent transcription of the "integrated" DNA of the >2n phagocytic cells into RNA and the translation of the latter into proteins produces a phenotype different from the phagocytic cells that have not phagocytosed the diseased cells (i.e., the =2n phagocytic cells). In other embodiments, the internalized disease-specific markers are not expressed by the >2n phagocytic cells. The markers may be translocated onto the membranes of the >2n phagocytic cells, or secreted out by the >2n phagocytic cells.

As used herein, a "profile" of a marker of a disease or condition can broadly refer to any information concerning the marker. This information can be either qualitative (e.g., presence or absence) or quantitative (e.g., levels, copy numbers, or dosages). In some particular examples, a profile of a marker can indicate the absence of this marker. The profile can be a nucleic acid (e.g., DNA or RNA) profile, a protein profile, a lipid profile, a carbohydrate profile, a metabolite profile, or a combination thereof. A "marker" as used herein generally refers to an analyte which is differentially detectable in phagocytes and is indicative of the presence of a disease or condition. An analyte is differentially detectable if it can be distinguished quantitatively or qualitatively in phagocytes.

The methods of this invention can be applied to various cardiovascular diseases or conditions. As used herein, "cardiovascular disease or condition" can refer to any disease, disorder, or condition affecting or associated with the cardiovascular system. Examples of cardiovascular diseases or conditions include, but are not limited to, myocardial infarction, coronary artery disease, percutaneous transluminal coronary angioplasty (PTCA), coronary artery bypass surgery (CABG), restenosis, peripheral arterial disease, stroke, abdominal aorta aneurysm, intracranial aneurysm, large artery atherosclerotic stroke, cardiogenic stroke, an early onset myocardial infarction, heart failure, pulmonary embolism, acute coronary syndrome (ACS), angina, cardiac hypertrophy, arteriosclerosis, myocarditis, pancarditis, endocarditis, hypertension, congestive heart failure, atherosclerosis, cerebrovascular disease, declining cardiac health, ischemic heart disease, pericarditis, cardiogenic shock, alcoholic cardiomyopathy, congenital heart disease, ischemic cardiomyopathy, hypertensive cardiomyopathy, valvular cardiomyopathy, inflammatory cardiomyopathy, cardiomyopathy secondary to a systemic metabolic disease, dilated cardiomyopathy, hypertrophic cardiomyopathy, arrhythmogenic right ventricular cardiomyopathy, restrictive cardiomyopathy, noncompaction cardiomyopathy, valvular heart disease, hypertensive heart disease, myocardial ischemic attack, unstable angina, myocardial rupture, cardiogenic shock, embolism, deep vein thrombosis, arrhythmia, arrhythmogenic right ventricular cardiomyopathy, diabetic cardiomyopathy, mitral regurgitation, mitral valve prolapse, peripheral vascular disease, artery disease, carotid artery disease, deep vein thrombosis, venous diseases, cerebrovascular disease, arterial aneurysm, left ventricular hypertrophy, hypertensive renal disease, hypertensive retinal disease, vasculitis, left main disease, arterial vascular disease, venous vascular disease, thrombosis of the microcirculation, a transient cerebrovascular accident, limb ischemia, aneurysm, thrombosis, superficial venous thrombosis, and deep venous thrombosis.

The methods of this invention can also be applied to the cardiovascular diseases or conditions disclosed in, for example, United States Patent Application Publications 20100068705, 20100009356, 20090305265, 20070148661, 20070141625, and International Patent Application Publications WO/2009/121152, WO/2009/097450, WO/2009/034470, WO/2009/014639, WO/2008/003826.

As used herein, "treating" a disease or condition refers to taking steps to obtain beneficial or desired results, including clinical results. Beneficial or desired clinical results include, but are not limited to, alleviation or amelioration of one or more symptoms associated with cardiovascular diseases or conditions.

As used herein, "administering" or "administration of" a compound or an agent to a subject can be carried out using one of a variety of methods known to those skilled in the art. For example, a compound or an agent can be administered, intravenously, arterially, intradermally, intramuscularly, intraperitonealy, intravenously, subcutaneously, ocularly, sublingually, orally (by ingestion), intranasally (by inhalation), intraspinally, intracerebrally, and transdermally (by absorbtion, e.g., through a skin duct). A compound or agent can also appropriately be introduced by rechargeable or biodegradable polymeric devices or other devices, e.g., patches and pumps, or formulations, which provide for the extended, slow, or controlled release of the compound or agent. Administering can also be performed, for example, once, a plurality of times, and/or over one or more extended periods. In some aspects, the administration includes both direct administration, including self-administration, and indirect administration, including the act of prescribing a drug. For example, as used herein, a physician who instructs a patient to self-administer a drug, or to have the drug administered by another and/or who provides a patient with a prescription for a drug is administering the drug to the patient. In some examples, a compound or an agent is administered orally, e.g., to a subject by ingestion, or intravenously, e.g., to a subject by injection. In some examples, the orally administered compound or agent is in an extended release or slow release formulation, or administered using a device for such slow or extended release.

In certain particular examples, markers used in the methods of invention are up-regulated or activated in the phagocytic cells compared to the non-phagocytic cells. In certain particular examples, markers used in the methods of invention are down-regulated or inhibited in the phagocytic cells compared to the non-phagocytic cells. In certain particular examples, markers used in the methods of invention are up-regulated or activated in the >2n phagocytic cells compared to the =2n phagocytic cells. In certain particular examples markers used in the methods of invention are down-regulated or inhibited in the >2n phagocytic cells compared to the =2n phagocytic cells. Different diseases or conditions can be associated with either up-regulation (or activation) or down-regulation (or inhibition) of different markers. As used herein, "up-regulation or up-regulated" can refer to an increase in expression levels (e.g., gene expression or protein expression), gene copy numbers, gene dosages, and other qualitative or quantitative detectable state of the markers. Similarly, "down-regulation or down-regulated" can refer to an increase in expression levels, gene copy numbers, gene dosages, and other qualitative or quantitative detectable state of the markers. As used herein, "activation or activated" can refer to an active state of the marker, e.g., a phosphorylation state, a DNA methylation state, or a DNA acetylation state. Similarly, "inhibition or inhibited" can refer to a repressed state or an inactivated state of the marker, e.g., a de-phosphorylation state, a ubiquitination state, a DNA de-methylation state.

In certain particular examples, methods of this invention also comprise at least one of the following steps before determination of various profiles: i) lysing the phagocytic cells and the non-phagocytic cells; ii) extracting cellular contents from the lysed phagocytic cells, the lysed non-phagocytic cells. Any known cell lysis and extraction methods can be used herein. In certain embodiments, the cellular contents of the phagocytic cells comprise various types of materials that they have engulfed, such as, viable diseased cells, dead diseased cells, apoptotic diseased cells, circulating tumor cells, infectious agents, fetal cells, trophoblasts, or fragments thereof. In certain examples, at least one or more markers of a cardiovascular disease or condition are present in the cellular contents of the phagocytic cells. In certain examples, there is no marker present in the cellular contents of the non-phagocytic cells.

In certain examples, methods of this invention also comprise at least one of the following steps before determination of various profiles: i) lysing the >2n phagocytic cells and the =2n phagocytic cells; and ii) extracting cellular contents from the lysed >2n phagocytic cells and the lysed =2n phagocytic cells. In certain examples, the cellular contents of the >2n phagocytic cells comprise various types of materials that they have engulfed, such as, viable diseased cells, dead diseased cells, apoptotic diseased cells, circulating tumor cells, infectious agents, fetal cells, trophoblasts, or fragments thereof. In certain examples, at least one or more markers of a cardiovascular disease or condition are present in the cellular contents of the >2n phagocytic cells. In certain examples, there is no marker present in the cellular contents of the =2n phagocytic cells.

In certain examples, methods of this invention further comprise comparing the identified difference of the disease or condition-specific markers to a repository of at least one markers known in the art. Such comparison can further confirm the presence of the disease or condition. In some embodiments, the repository of the known markers can be obtained by data mining. The term "data mining", as used herein, refers to a process of finding new data patterns, relations, or correlations derived from the known data of the databases and of extracting practicable information in the future. Typically a computer-based system can be trained on data to perform the data mining, e.g., to classify the input data and then subsequently used with new input data to make decisions based on the training data. These systems include, but are not limited, expert systems, fuzzy logic, nonlinear regression analysis, multivariate analysis, decision tree classifiers, and Bayesian belief networks.

In certain embodiments, the phagocytic cells (e.g., the >2n and the =2n subpopulations) and the non-phagocytic cells are isolated from a bodily fluid sample, tissues, or cells. Exemplar bodily fluid sample can be whole blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, maternal urine, maternal saliva, placental sample, fetal blood, lavage and cervical vaginal fluid, interstitial fluid, or ocular fluid. In some examples, the phagocytic cells (e.g., the >2n and the =2n subpopulations) and the non-phagocytic cells are isolated from white blood cells. In certain examples, the >2n phagocytic cells and the =2n phagocytic cells are separated from a population of phagocytic cells.

In the present methods, cell separation/isolation/purification methods are used to isolate populations of cells from bodily fluid sample, cells, or tissues of a subject. A skilled worker can use any known cell separation/isolation/purification techniques to isolate phagocytic cells or non-phagocytic cells from a bodily fluid, or to separate phagocytic cells from non-phagocytic cells, or to separate >2n phagocytic cells from =2n phagocytic cells. Exemplar techniques include, but are not limited to, using antibodies, flow cytometry, fluorescence activated cell sorting, filtration, gradient-based centrifugation, elution, microfluidics, magnetic separation technique, fluorescent-magnetic separation technique, nanostructure, quantum dots, high throughput microscope-based platform, or a combination thereof.

In certain examples, the phagocytic cells and the non-phagocytic cells are isolated by using a product secreted by the phagocytic cells. In certain examples, the phagocytic cells and the non-phagocytic cells are isolated by using a cell surface target (e.g., receptor protein) on the surface of phagocytic cells. In some examples, the cell surface target is a protein that has been engulfed by the phagocytic cells. In some examples, the cell surface target is expressed by the phagocytic cells on their plasma membranes. In some examples, the cell surface target is an exogenous protein that is translocated on the plasma membranes, but not expressed by the phagocytic cells. In some examples, the cell surface target is a marker of the disease or condition to be detected.

In certain examples, the >2n phagocytic cells and the =2n phagocytic cells are isolated by using a product secreted by the >2n phagocytic cells. In certain examples, the >2n phagocytic cells and the =2n phagocytic cells are isolated by using a cell surface target (e.g., receptor protein) on the surface of phagocytic cells. In some examples, the cell surface target is a protein that has been engulfed by the >2n phagocytic cells. In some examples, the cell surface target is expressed by the >2n phagocytic cells on their plasma membranes. In some examples, the cell surface target is an exogenous protein that is translocated on the plasma membranes, but not expressed by the >2n phagocytic cells. In some examples, the cell surface target is a marker of the disease or condition to be detected.

In certain aspects of the methods described herein, analytes include nucleic acids, proteins, lipids, carbohydrates, metabolites, or any combinations of these. In certain aspects of the methods described herein, markers include nucleic acids, proteins, lipids, carbohydrates, metabolites, or any combinations of these. As used herein, the term "nucleic acid" is intended to include DNA molecules (e.g., cDNA or genomic DNA), RNA molecules (e.g., mRNA), DNA-RNA hybrids, and analogs of the DNA or RNA generated using nucleotide analogs. The nucleic acid molecule can be a nucleotide, oligonucleotide, double-stranded DNA, single-stranded DNA, multi-stranded DNA, complementary DNA, genomic DNA, noncoding DNA, messenger RNA (mRNAs), microRNA (miRNAs), small nucleolar RNA (snoRNAs), ribosomal RNA (rRNA), transfer RNA (tRNA), small interfering RNA (siRNA), heterogeneous nuclear RNAs (hnRNA), or small hairpin RNA (shRNA).

As used herein, the term "amino acid" includes organic compounds containing both a basic amino group and an acidic carboxyl group. Included within this term are natural amino acids (e.g., L-amino acids), modified and unusual amino acids (e.g., D-amino acids and β-amino acids), as well as amino acids which are known to occur biologically in free or combined form but usually do not occur in proteins. Natural protein occurring amino acids include alanine, arginine, asparagine, aspartic acid, cysteine, glutamic acid, glutamine, glycine, histidine, isoleucine, leucine, lysine, methionine, phenylalanine, serine, threonine, tyrosine, tryptophan, proline, and valine. Natural non-protein amino acids include arginosuccinic acid, citrulline, cysteine sulfuric acid, 3,4-dihydroxyphenylalanine, homocysteine, homoserine, ornithine, 3-monoiodotyrosine, 3,5-diiodotryosine, 3, 5, 5-triiodothyronine, and 3,3',5,5'- tetraiodothyronine. Modified or unusual amino acids include D-amino acids, hydroxylysine, 4-hydroxyproline, N-Cbz-protected amino acids, 2,4-diaminobutyric acid, homoarginine, norleucine, N-methylaminobutyric acid, naphthylalanine, phenylglycine, .alpha.-phenylproline, tert-leucine, 4-aminocyclohexylalanine, N-methyl-norleucine, 3 ,4-dehydroproline, N,N-dimethylaminoglycine, N-methylaminoglycine, 4-aminopiperidine-4-carboxylic acid, 6-aminocaproic acid, trans-4-(aminomethyl)-cyclohexanecarboxylic acid, 2-, 3-, and 4-(aminomethyl)- benzoic acid, 1-aminocyclopentanecarboxylic acid, 1-aminocyclopropanecarboxylic acid, and 2-benzyl-5-aminopentanoic acid.

As used herein, the term "peptide" includes compounds that consist of two or more amino acids that are linked by means of a peptide bond. Peptides may have a molecular weight of less than 10,000 Daltons, less than 5,000 Daltons, or less than 2,500 Daltons. The term "peptide" also includes compounds containing both peptide and non-peptide components, such as pseudopeptide or peptidomimetic residues or other non-amino acid components. Such compounds containing both peptide and non-peptide components may also be referred to as a "peptide analog."

As used herein, the term "protein" includes compounds that consist of amino acids arranged in a linear chain and joined together by peptide bonds between the carboxyl and amino groups of adjacent amino acid residues. Proteins used in methods of the invention include, but are not limited to, amino acids, peptides, antibodies, antibody fragments, cytokines, lipoproteins, or glycoproteins.

As used herein, the term "antibody" includes polyclonal antibodies, monoclonal antibodies (including full length antibodies which have an immunoglobulin Fc region), antibody compositions with polyepitopic specificity, multispecific antibodies (e.g., bispecific antibodies, diabodies, and single-chain molecules, and antibody fragments (*e.g.,* Fab or F(ab')₂, and Fv). For the structure and properties of the different classes of antibodies, see e.g., Basic and Clinical Immunology, 8th Edition, Daniel P. Sties, Abba I. Terr and Tristram G. Parsolw (eds), Appleton & Lange, Norwalk, Conn., 1994, page 71 and Chapter 6.

As used herein, the term "cytokine" refers to a secreted protein or active fragment or mutant thereof that modulates the activity of cells of the immune system. Examples of cytokines include, without limitation, interleukins, interferons, chemokines, tumor necrosis factors, colony-stimulating factors for immune cell precursors, and the like.

As used herein, the term "lipoprotein" includes negatively charged compositions that comprise a core of hydrophobic cholesteryl esters and triglyceride surrounded by a surface layer of amphipathic phospholipids with which free cholesterol and apolipoproteins are associated. Lipoproteins may be characterized by their density (e.g. very-low-density lipoprotein (VLDL), low-density lipoprotein (LDL) and high density lipoprotein (HDL)), which is determined by their size, the relative amounts of lipid and protein. Lipoproteins may also be characterized by the presence or absence of particular modifications (e.g. oxidization, acetylation, or glycation).

As used herein, the term "glycoprotein" includes glycosides which have one or more oligo- or polysaccharides covalently attached to a peptide or protein. Exemplary glycoproteins can include, without limitation, immunoglobulins, members of the major histocompatibility complex, collagens, mucins, glycoprotein IIb/IIIa, glycoprotein-41 (gp41) and glycoprotein-120 (gp12), follicle-stimulating hormone, alpha-fetoprotein, erythropoietin, transferrins, alkaline phosphatase, and lectins.

As used herein, the term "lipid" includes synthetic or naturally-occurring compounds which are generally amphipathic and biocompatible. Lipids typically comprise a hydrophilic component and a hydrophobic component. Exemplary lipids include, but are not limited to fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, sulfatide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin,, gangliosides, plasmalogen, sulfatide, ceramide, low density lipoproteins (LDLs), very low density lipoproteins (VLDLs), high density lipoproteins (HDLs), sphingoid base-1-phosphates or derivatives thereof.

As used herein, the term "carbohydrate" includes, but is not limited to, compounds that contain oxygen, hydrogen and carbon atoms, typically (CH₂O)ₙ wherein n is an integer. Exemplary carbohydrates include, but are not limited to, monosaccharides, disaccharides, polysaccharides, or oligosaccharides.

As used herein, the term "metabolite" includes any molecule used in metabolism. Metabolites can be products, substrates, or intermediates in metabolic processes. Included within this term are primary metabolites, secondary metabolites, organic metabolites, or inorganic metabolites. Metabolites include, without limitation, amino acids, peptides, acylcarnitines, monosaccharides, lipids and phospholipids, prostaglandins, hydroxyeicosatetraenoic acids, hydroxyoctadecadienoic acids, steroids, bile acids, and glycolipids and phospholipids. Exemplary metabolites can be sphingolipids, glycosphingolipids, sphingosine, ceramide, sphingomyelin, sphingosylphosphorylcholin, dihydrosphingosine, phoshatidylcholine, phosphatidylinositol, phosphatidylserine, lysophoshatidylcholine, lysophosphatidylinositol, lysophosphatidylserine, plasmenylphoshatidylcholine, plasmanylphoshatidylcholine, proteinogenic amino acids, Alanine, Aspartic acid, Glutamic acid, Phenylalanine, Glycine, Histidine, Leucine, Isoleucine, Lysine, Methionine, Proline, Arginine, Serine, Threonine, Valine, Tryptophan, Tyrosine, asymmetrical dimethyl arginine, symmetrical dimethyl arginine, Glutamine, Asparagine, Nitrotyrosine, Hydroxyproline, Kynurenine, 3-Hydroxy kynurenine, non-proteinogenic amino acids, Ornithine, Citrulline, acylcarnitines, carnitine, free carnitine, acylcarnitine, hydroxylacylcarnitine, dicarboxylacylcarnitines, reducing monosaccharides, hexose, pentose, deoxyhexose, creatinine, creatine, spermidine spermine, putrescine, dopamine, serotonin, prostaglandins, hydoxyeicosatetraeneoic acid, Hydroxyoctadecadienoic acid, leukatrienes, thromboxanes, bile acids, sterols, cholesterols, vitamins and cofactors, drugs, and drug metabolites.

In some embodiments of the invention, profiles of at least one or more markers of a cardiovascular disease or condition are compared. This comparison can be quantitative or qualitative. Quantitative measurements can be taken using any of the assays described herein. For example, sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

Quantitative comparisons can include statistical analyses such as t-test, ANOVA, Krustal-Wallis, Wilcoxon, Mann-Whitney, and odds ratio. Quantitative differences can include differences in the levels of markers between profiles or differences in the numbers of markers present between profiles, and combinations thereof. Examples of levels of the markers can be, without limitation, gene expression levels, nucleic acid levels, protein levels, lipid levels, and the like. Qualitative differences can include, but are not limited to, activation and inactivation, protein degradation, nucleic acid degradation, and covalent modifications.

In certain embodiments of the invention, the profile is a nucleic acid profile, a protein profile, a lipid profile, a carbohydrate profile, a metabolite profile, or a combination thereof. The profile can be qualitatively or quantitatively determined.

A nucleic acid profile can be, without limitation, a genotypic profile, a single nucleotide polymorphism profile, a gene mutation profile, a gene copy number profile, a DNA methylation profile, a DNA acetylation profile, a chromosome dosage profile, a gene expression profile, or a combination thereof.

The nucleic acid profile can be determined by any methods known in the art to detect genotypes, single nucleotide polymorphisms, gene mutations, gene copy numbers, DNA methylation states, DNA acetylation states, chromosome dosages. Exemplar methods include, but are not limited to, polymerase chain reaction (PCR) analysis, sequencing analysis, electrophoretic analysis, restriction fragment length polymorphism (RFLP) analysis, Northern blot analysis, quantitative PCR, reverse-transcriptase-PCR analysis (RT-PCR), allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), surface plasmon resonance, Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, bisulfite-driven conversion of non-methylated cytosine to uracil, methyl-binding PCR analysis, or a combination thereof.

As used herein, the term "sequencing" is used in a broad sense and refers to any technique known in the art that allows the order of at least some consecutive nucleotides in at least part of a nucleic acid to be identified, including without limitation at least part of an extension product or a vector insert. Exemplar sequencing techniques include direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof. In some embodiments, sequencing comprises an detecting the sequencing product using an instrument, for example but not limited to an ABI PRISM® 377 DNA Sequencer, an ABI PRISM® 310, 3100, 3100-Avant, 3730, or 373OxI Genetic Analyzer, an ABI PRISM® 3700 DNA Analyzer, or an Applied Biosystems SOLiD™ System (all from Applied Biosystems), a Genome Sequencer 20 System (Roche Applied Science), or a mass spectrometer. In certain embodiments, sequencing comprises emulsion PCR. In certain embodiments, sequencing comprises a high throughput sequencing technique, for example but not limited to, massively parallel signature sequencing (MPSS).

In further embodiments of the invention, a protein profile can be a protein expression profile, a protein activation profile, or a combination thereof. In some embodiments, a protein activation profile can comprise determining a phosphorylation state, an ubiquitination state, a myristoylation state, or a conformational state of the protein.

A protein profile can be detected by any methods known in the art for detecting protein expression levels, protein phosphorylation state, protein ubiquitination state, protein myristoylation state, or protein conformational state. In some embodiments, a protein profile can be determined by an immunohistochemistry assay, an enzyme-linked immunosorbent assay (ELISA), in situ hybridization, chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microscopy, microfluidic chip-based assays, surface plasmon resonance, sequencing, Western blotting assay, or a combination thereof.

In some embodiments of the invention, a lipid profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof. Further methods for analyzing lipid content in a biological sample are known in the art (See, e.g., Kang et al. (1992) Biochim. Biophys. Acta. 1128:267; Weylandt et al. (1996) Lipids 31 :977; J. Schiller et al. (1999) Anal. Biochem. 267:46; Kang et al. (2001) Proc. Natl. Acad. Sci. USA 98:4050; Schiller et al. (2004) Prog. Lipid Res. 43:499). One exemplary method of lipid analysis is to extract lipids from a biological sample (e.g. using chloroform-methanol (2:1, vol/vol) containing 0.005% butylated hydroxytoluene (BHT, as an antioxidant)), prepare fatty acid methyl esters (e.g., using 14% BF3-methanol reagent), and quantify the fatty acid methyl esters (e.g., by HPLC, TLC, by gas chromatography-mass spectroscopy using commercially available gas chromatographs, mass spectrometers, and/or combination gas chromatograph/mass spectrometers). Fatty acid mass is determined by comparing areas of various analyzed fatty acids to that of a fixed concentration of internal standard.

In some embodiments of the invention, a carbohydrate profile can be determined by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

In some particular examples of the invention, a metabolite profile can be determind by chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assay, detection of fluorescence, detection of chemiluminescence, or a combination thereof.

As used herein, the "difference" between different profiles detected by the methods of this invention can refer to different gene copy numbers, different DNA, RNA, protein, lipid, or carbohydrate expression levels, different DNA methylation states, different DNA acetylation states, and different protein modification states. The difference can be a difference greater than 1 fold. In some particular examples, the difference is a 1.05-fold, 1.1-fold, 1.2-fold, 1.3-fold, 1.4-fold, 1.5-fold, 2-fold, 2.5-fold, 3-fold, 4-fold, 5-fold, 6-fold, 7-fold, 8-fold, 9-fold, or 10-fold difference. In some particular examples, the difference is any fold difference between 1-10, 2-10, 5-10, 10-20, or 10-100 folds.

A general principle of assays to detect markers involves preparing a sample or reaction mixture that may contain the marker (e.g., one or more of DNA, RNA, protein, polypeptide, carbohydrate, lipid, metabolite, and the like) and a probe under appropriate conditions and for a time sufficient to allow the marker and probe to interact and bind, thus forming a complex that can be removed and/or detected in the reaction mixture. These assays can be conducted in a variety of ways.

For example, one method to conduct such an assay would involve anchoring the marker or probe onto a solid phase support, also referred to as a substrate, and detecting target marker/probe complexes anchored on the solid phase at the end of the reaction. In one particular example of such a method, a sample from a subject, which is to be assayed for presence and/or concentration of marker, can be anchored onto a carrier or solid phase support. In another particular example, the reverse situation is possible, in which the probe can be anchored to a solid phase and a sample from a subject can be allowed to react as an unanchored component of the assay.

There are many established methods for anchoring assay components to a solid phase. These include, without limitation, marker or probe molecules which are immobilized through conjugation of biotin and streptavidin. Such biotinylated assay components can be prepared from biotin-NHS(N-hydroxy-succinimide) using techniques known in the art (e.g., biotinylation kit, Pierce Chemicals, Rockford, IL), and immobilized in the wells of streptavidin-coated 96 well plates (Pierce Chemical). In certain particular examples, the surfaces with immobilized assay components can be prepared in advance and stored.

Other suitable carriers or solid phase supports for such assays include any material capable of binding the class of molecule to which the marker or probe belongs. Well known supports or carriers include, but are not limited to, glass, polystyrene, nylon, polypropylene, nylon, polyethylene, dextran, amylases, natural and modified celluloses, polyacrylamides, gabbros, and magnetite.

In order to conduct assays with the above mentioned approaches, the non-immobilized component is added to the solid phase upon which the second component is anchored. After the reaction is complete, uncomplexed components may be removed (e.g., by washing) under conditions such that any complexes formed will remain immobilized upon the solid phase. The detection of marker/probe complexes anchored to the solid phase can be accomplished in a number of methods outlined herein.

In certain particular examples, the probe, when it is the unanchored assay component, can be labeled for the purpose of detection and readout of the assay, either directly or indirectly, with detectable labels discussed herein and which are well-known to one skilled in the art.

It is also possible to directly detect marker/probe complex formation without further manipulation or labeling of either component (marker or probe), for example by utilizing the technique of fluorescence energy transfer (see, for example, U.S. Patent Nos. 5,631,169 and 4,868,103). A fluorophore label on the first, 'donor' molecule is selected such that, upon excitation with incident light of appropriate wavelength, its emitted fluorescent energy will be absorbed by a fluorescent label on a second 'acceptor' molecule, which in turn is able to fluoresce due to the absorbed energy. Alternately, the 'donor' protein molecule may simply utilize the natural fluorescent energy of tryptophan residues. Labels are chosen that emit different wavelengths of light, such that the 'acceptor' molecule label may be differentiated from that of the 'donor'. Since the efficiency of energy transfer between the labels is related to the distance separating the molecules, spatial relationships between the molecules can be assessed. In a situation in which binding occurs between the molecules, the fluorescent emission of the 'acceptor' molecule label in the assay should be maximal. An FET binding event can be conveniently measured through standard fluorometric detection means well known in the art (e.g., using a fluorimeter).

In another embodiment, determination of the ability of a probe to recognize a marker can be accomplished without labeling either assay component (probe or marker) by utilizing a technology such as real-time Biomolecular Interaction Analysis (BIA) (see, e.g., Sjolander, S. and Urbaniczky, C, 1991, Anal. Chem. 63:2338 2345 and Szabo et al, 1995, Curr. Opin. Struct. Biol. 5:699 705). As used herein, "BIA" or "surface plasmon resonance" is a technology for studying biospecific interactions in real time, without labeling any of the interactants (e.g., BIAcore). Changes in the mass at the binding surface (indicative of a binding event) result in alterations of the refractive index of light near the surface (the optical phenomenon of surface plasmon resonance (SPR)), resulting in a detectable signal which can be used as an indication of real-time reactions between biological molecules.

Alternatively, in another example, analogous diagnostic and prognostic assays can be conducted with marker and probe as solutes in a liquid phase. In such an assay, the complexed marker and probe are separated from uncomplexed components by any of a number of standard techniques, including but not limited to: differential centrifugation, chromatography, electrophoresis and immunoprecipitation. In differential centrifugation, marker/probe complexes may be separated from uncomplexed assay components through a series of centrifugal steps, due to the different sedimentation equilibria of complexes based on their different sizes and densities (see, for example, Rivas and Minton (1993) Trends Biochem. Sci. 18:284). Standard chromatographic techniques may also be utilized to separate complexed molecules from uncomplexed ones. For example, gel filtration chromatography separates molecules based on size, and through the utilization of an appropriate gel filtration resin in a column format, for example, the relatively larger complex may be separated from the relatively smaller uncomplexed components. Similarly, the relatively different charge properties of the marker/probe complex as compared to the uncomplexed components may be exploited to differentiate the complex from uncomplexed components, for example through the utilization of ion-exchange chromatography resins. Such resins and chromatographic techniques are well known to one skilled in the art (see, e.g., Heegaard (1998) J. MoI. Recognit. 11 :141; Hage and Tweed (1997) J. Chromatogr. B. Biomed. Sci. Appl. 12:499). Gel electrophoresis may also be employed to separate complexed assay components from unbound components (see, e.g., Ausubel et al, ed., Current Protocols in Molecular Biology, John Wiley & Sons, New York, 1987 1999). In this technique, protein or nucleic acid complexes are separated based on size or charge, for example. In order to maintain the binding interaction during the electrophoretic process, non-denaturing gel matrix materials and conditions in the absence of reducing agent are typically preferred. Appropriate conditions to the particular assay and components thereof will be well known to one skilled in the art.

In certain particular examples, the level of mRNA corresponding to the marker can be determined either by *in situ* and/or by *in vitro* formats in a biological sample using methods known in the art. Many expression detection methods use isolated RNA. For in vitro methods, any RNA isolation technique that does not select against the isolation of mRNA can be utilized for the purification of RNA from blood cells (see, e.g., Ausubel et al, ed., Current Protocols in Molecular Biology, John Wiley & Sons, New York 1987 1999). Additionally, large numbers of cells and/or samples can readily be processed using techniques well known to those of skill in the art, such as, for example, the single-step RNA isolation process of Chomczynski (1989, U.S. Patent No. 4,843,155).

Isolated mRNA can be used in hybridization or amplification assays that include, but are not limited to, Southern or Northern analyses, polymerase chain reaction analyses and probe arrays. In certain examples, a diagnostic method for the detection of mRNA levels involves contacting the isolated mRNA with a nucleic acid molecule (probe) that can hybridize to the mRNA encoded by the gene being detected. The nucleic acid probe can be, for example, a full-length cDNA, or a portion thereof, such as an oligonucleotide of at least 7, 15, 30, 50, 100, 250 or 500 nucleotides in length and sufficient to specifically hybridize under stringent conditions to an mRNA or genomic DNA encoding a marker of the present invention. Other suitable probes for use in the diagnostic assays of the invention are described herein. Hybridization of an mRNA with the probe indicates that the marker in question is being expressed.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe(s), for example, in a gene chip array. A skilled artisan can readily adapt known mRNA detection methods for use in detecting the level of mRNA encoded by the markers of the present invention.

An alternative method for determining the level of mRNA corresponding to a marker of the present invention in a sample involves the process of nucleic acid amplification, e.g., by RT-PCR (the experimental embodiment set forth in U.S. Patent Nos. 4,683,195 and 4,683,202), COLD-PCR (Li et al. (2008) Nat. Med. 14:579), ligase chain reaction (Barany, 1991, Proc. Natl. Acad. Sci. USA, 88:189), self sustained sequence replication (Guatelli et al., 1990, Proc. Natl. Acad. Sci. USA 87:1874), transcriptional amplification system (Kwoh et al. (1989) Proc. Natl. Acad. Sci. USA 86:1173), Q- Beta Replicase (Lizardi et al. (1988) Bio/Technology 6:1197), rolling circle replication (U.S. Patent No. 5,854,033) or any other nucleic acid amplification method, followed by the detection of the amplified molecules using techniques well known to those of skill in the art. These detection schemes are especially useful for the detection of nucleic acid molecules if such molecules are present in very low numbers. As used herein, amplification primers are defined as being a pair of nucleic acid molecules that can anneal to 5' or 3' regions of a gene (plus and minus strands, respectively, or vice-versa) and contain a short region in between. In general, amplification primers are from about 10 to 30 nucleotides in length and flank a region from about 50 to 200 nucleotides in length. Under appropriate conditions and with appropriate reagents, such primers permit the amplification of a nucleic acid molecule comprising the nucleotide sequence flanked by the primers.

For *in situ* methods, mRNA does not need to be isolated from the sample (*e.g.,* a bodily fluid (*e.g.,* blood cells)) prior to detection. In such methods, a cell or tissue sample is prepared/processed using known histological methods. The sample is then immobilized on a support, typically a glass slide, and then contacted with a probe that can hybridize to mRNA that encodes the marker.

As an alternative to making determinations based on the absolute expression level of the marker, determinations may be based on the normalized expression level of the marker. Expression levels are normalized by correcting the absolute expression level of a marker by comparing its expression to the expression of a gene that is not a marker, *e.g.,* a housekeeping gene that is constitutively expressed. Suitable genes for normalization include housekeeping genes such as the actin gene, or epithelial cell- specific genes. This normalization allows the comparison of the expression level in a patient sample from one source to a patient sample from another source, e.g., to compare a phagocytic blood cell from an individual to a non-phagocytic blood cell from the individual.

In one embodiment of this invention, a protein or polypeptide corresponding to a marker is detected. In certain particular examples, an agent for detecting a protein or polypeptide can be an antibody capable of binding to the polypeptide, such as an antibody with a detectable label. As used herein, the term "labeled," with regard to a probe or antibody, is intended to encompass direct labeling of the probe or antibody by coupling (i.e., physically linking) a detectable substance to the probe or antibody, as well as indirect labeling of the probe or antibody by reactivity with another reagent that is directly labeled. Examples of indirect labeling include detection of a primary antibody using a fluorescently labeled secondary antibody and end-labeling of a DNA probe with biotin such that it can be detected with fluorescently labeled streptavidin. Antibodies can be polyclonal or monoclonal. An intact antibody, or a fragment thereof (*e.g.,* Fab or F(ab')2) can be used. In one format, antibodies, or antibody fragments, can be used in methods such as Western blots or immunofluorescence techniques to detect the expressed proteins. In such uses, it is generally preferable to immobilize either the antibody or proteins on a solid support. Suitable solid phase supports or carriers include any support capable of binding an antigen or an antibody. Well known supports or carriers include glass, polystyrene, polypropylene, polyethylene, dextran, nylon, amylases, natural and modified celluloses, polyacrylamides, gabbros, magnetite and the like.

A variety of formats can be employed to determine whether a sample contains a protein that binds to a given antibody. Examples of such formats include, but are not limited to, competitive and non-competitive immunoassay, enzyme immunoassay (EIA), radioimmunoassay (RIA), antigen capture assays, two-antibody sandwich assays, Western blot analysis, enzyme linked immunoabsorbant assay (ELISA), a planar array, a colorimetric assay, a chemiluminescent assay, a fluorescent assay, and the like. Immunoassays, including radioimmmunoassays and enzyme- linked immunoassays, are useful in the methods of the present invention. A skilled artisan can readily adapt known protein/antibody detection methods for use in determining whether cells (e.g., bodily fluid cells such as blood cells) express a marker of the present invention.

One skilled in the art will know many other suitable carriers for binding antibody or antigen, and will be able to adapt such support for use with the present invention. For example, protein isolated from cells (e.g., bodily fluid cells such as blood cells) can be run on a polyacrylamide gel electrophoresis and immobilized onto a solid phase support such as nitrocellulose. The support can then be washed with suitable buffers followed by treatment with the detectably labeled antibody. The solid phase support can then be washed with the buffer a second time to remove unbound antibody. The amount of bound label on the solid support can then be detected by conventional means.

In certain particular examples, assays are provided for diagnosis, prognosis, assessing the risk of developing a disease, assessing the efficacy of a treatment, monitoring the progression or regression of a disease, and identifying a compound capable of ameliorating or treating a disease. An exemplary method for these methods involves obtaining a bodily fluid sample from a test subject and contacting the bodily fluid sample with a compound or an agent capable of detecting one or more of the markers of the disease or condition, *e.g.,* marker nucleic acid (*e.g.,* mRNA, genomic DNA), marker peptide (*e.g.,* polypeptide or protein), marker lipid (*e.g.,* cholesterol), or marker metabolite (*e.g.,* creatinine) such that the presence of the marker is detected in the biological sample. In one particular example, an agent for detecting marker mRNA or genomic DNA is a labeled nucleic acid probe capable of hybridizing to marker mRNA or genomic DNA. The nucleic acid probe can be, for example, a full-length marker nucleic acid or a portion thereof. Other suitable probes for use in the diagnostic assays of the invention are described herein.

As used herein, a compound capable of ameliorating or treating a cardiovascular disease or condition can include, without limitations, any substance that can improve symptoms or prognosis, prevent progression of the disease or condition, promote regression of the disease or condition, or eliminate the disease or condition.

In yet another aspect, a method for identifying a compound capable of ameliorating or treating a cardiovascular disease or condition in a subject is provided, said method comprising: a) determining a first profile of one or more markers of the disease or condition from a population of>2n phagocytic cells from the subject before administering the compound to the subject; determining a second profile of at least one of the one or more markers from a population of =2n phagocytic cells from the subject before administering the compound to the subject; identifying a first difference between the first and second profiles of at least one or more of said markers; b) determining a third profile of the one or more markers from a population of>2n phagocytic cells from the subject after the administration of the compound; determining a fourth profile of at least one of the one or more markers from a population of =2n phagocytic cells from the subject after the administration of the compound; identifying a second difference between the third and fourth profiles of at least one or more of said markers; c) identifying a difference between the first difference and the second difference, wherein the identified difference indicates that the compound is capable of ameliorating or treating said disease or condition in the subject.

In yet another aspect, a method for identifying one or more markers for a cardiovascular disease or condition is provided, said method comprising: a) determining a first profile of analytes from phagocytic cells from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from phagocytic cells from a control subject not having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. Optionally, this method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, a method for identifying one or more markers of a cardiovascular disease or condition is provided, said method comprising: a) determining a first profile of analytes from phagocytic cells from a subject having said disease or condition; determining a second profile of analytes from phagocytic cells from a control subject not having said disease or condition; identifying a first set of differences between the first and second profiles, wherein the first set of differences is specific to the first profile relative to the second profile; b) determining a third profile of analytes from non-phagocytic cells from the subject having said disease or condition; determining a fourth profile of analytes from non-phagocytic cells from the control subject not having said disease or condition; identifying a second set of differences between the third and fourth profiles, wherein the second set of differences is specific to the third profile relative to the fourth profile; c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition in the subject having said disease or condition; obtaining a sixth profile of analytes from cells or tissues not affected by said disease or condition in the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

In yet another aspect, a method for identifying one or more markers of a cardiovascular disease or condition is provided, said method comprising: a) determining a first profile of analytes from phagocytic cells from a subject having said disease or condition; obtaining a second profile of analytes from phagocytic cells from a control subject not having said disease or condition by data mining; identifying a first set of differences between the first and second profiles, wherein the first set of differences is specific to the first profile relative to the second profile; b) determining a third profile of analytes from non-phagocytic cells from the subject having said disease or condition; obtaining a fourth profile of analytes from non-phagocytic cells from a control subject not having said disease or condition by data mining; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises d) obtaining a fifth profile of analytes from cells or tissues affected by said disease or condition by data mining; obtaining a sixth profile of analytes from cells or tissues not affected by said disease or condition by data mining; identifying a third set of differences between the fifth and sixth profiles, wherein the third set of differences is specific to the fifth profile relative to the sixth profile; and e) identifying at least one of the one or more markers of c) present in the third set of differences.

In yet another aspect, a method for identifying one or more markers of a cardiovascular disease or condition is provided, said method comprising: a) determining a first profile of analytes from phagocytic cells from a subject having said disease or condition; determining a second profile of analytes from non-phagocytic cells from the subject having said disease or condition; identifying a first set of differences between the first and second profiles, wherein the first set of differences is specific to the first profile relative to the second profile; b) determining a third profile of analytes from cells or tissues affected by said disease or condition from the subject having said disease or condition; determining a fourth profile of analytes from cells or tissues not affected by said disease or condition from the subject having said disease or condition; identifying a second set of differences between the third and fourth profiles, wherein the second set of differences is specific to the third profile relative to the fourth profile; c) identifying one or more analytes present in both the first set of differences and the second set of differences, the identified analytes being markers of said disease or condition. And optionally, the method further comprises d) determining a fifth profile of analytes from phagocytic cells from a control subject not having said disease or condition; identifying a third set of differences between the first and fifth profiles, wherein the third set of differences is specific to the first profile relative to the fifth profile; e) identifying at least one of the one or more markers of c) present in the third set of differences.

In yet another aspect, a method for identifying one or more markers of a cardiovascular disease or condition is provided, said method comprising: a) determining a first profile of analytes from >2n phagocytic cells from a subject having said disease or condition; determining a second profile of analytes from =2n phagocytic cells from the subject having said disease or condition;
identifying a first set of differences between the first and second profiles, wherein the first set of differences is specific to the first profile relative to the second profile; b) determining a third profile of analytes from >2n phagocytic cells from a control subject not having said disease or condition; determining a fourth profile of analytes from =2n phagocytic cells from the control subject not having said disease or condition; identifying a second set of differences between the third and fourth profiles, wherein the second set of differences is specific to the third profile relative to the fourth profile; and c) identifying one or more analytes specific to the first set of differences relative to the second set of differences, the identified analytes being markers of said disease or condition. And optionally, the method further comprises: d) obtaining a fifth profile of analytes from cells or tissues affected by said disease or condition from the subject having said disease or condition; obtaining a sixth profile of analytes from cells or tissues not affected by said disease or condition from the subject having said disease or condition; identifying a third set of differences between the fifth and sixth profiles, wherein the third set of differences is specific to the fifth profile relative to the sixth profile; and e) identifying at least one of the one or more markers of c) present in the third set of differences.

An exemplary method for detecting the presence or absence of an analyte (*e.g.,* DNA, RNA, protein, polypeptide, carbohydrate, lipid or the like) corresponding to a marker in a biological sample involves obtaining a bodily fluid sample (*e.g.,* blood) from a test subject and contacting the bodily fluid sample with a compound or an agent capable of detecting one or more markers. Detection methods described herein can be used to detect one or more markers in a biological sample *in vitro* as well as *in vivo.* For example, *in vitro* techniques for detection of mRNA include Northern hybridizations and in situ hybridizations. *In vitro* techniques for detection of a polypeptide corresponding to a marker include enzyme linked immunosorbent assays (ELISAs), Western blots, immunoprecipitations and immunofluorescence. *In vitro* techniques for detection of genomic DNA include Southern hybridizations. Furthermore, *in vivo* techniques for detection of a polypeptide corresponding to a marker include introducing into a subject a labeled antibody directed against the polypeptide. For example, the antibody can be labeled with a radioactive marker whose presence and location in a subject can be detected by standard imaging techniques. Because each marker is also an analyte, any method described herein to detect the presence or absence of a marker can also be used to detect the presence or absence of an analyte.

The marker that is useful in the methods of the invention includes those disclosed in, for example, United States Patents 7,670,769, 7,445,886, 7,432,107, 7,157,235, and 7,009,038, United States Patent Application Publications 20100167320, 20100112587, 20100098705, 20100068705, 20100009356, 20090305265, 20100124746, 20100092983, 20070148661, 20070141625, 20100120050, 20090155230, and 20090274709, and International Patent Application Publications WO/2009/121152, WO/2009/121951, WO/2009/097450, WO/2009/092382, WO/2009/075579, WO/2009/058168, WO/2009/053523, WO/2009/034470, WO/2009/032722, WO/2009/014639, WO/2009/003142, WO/2010/041046, WO/2007/131345, WO/2008/003826, and WO/2009/075566.

The marker that is useful in the methods of the invention can also include those markers disclosed in, for example, RS Vasan, Circulation (2006), 113:2335-2362; RC Becker, European Heart Journal (2005), 26:421-422; MP Bonaca and DA Morrow, Clinical Chemistry (2008) 54:1424-1431; FO Wood and JA de Lemos, Curr Cardiol Rep (2008), 10:319-326; A Palazzuoli et al., Minerva Cardioangiol (2007) 55:491-496, and by the American Association for Clinical Chemistry (Lab Tests Online, labtestsonline.org/understanding/analytes/cardiac_biomarkers/glance-2.html).

The marker that is useful in the methods of the invention can include any mutation in any one of the above-identified markers. Mutation sites and sequences can be identified, for example, by databases or repositories of such information, *e.g.,* The Human Gene Mutation Database (www.hgmd.cf.ac.uk), the Single Nucleotide Polymorphism Database (dbSNP, www.ncbi.nlm.nih.gov/projects/SNP), and the Online Mendelian Inheritance in Man (OMIM) website (www.ncbi.nlm.nih.gov/omim).

The marker that is useful in the methods of the invention can include any marker that is known to be associated with a cardiovascular disease or condition.

Kits that comprise marker detection agents that detect at least one or more of the markers identified by the methods of this invention are provided herein. Methods of treating or preventing a cardiovascular disease or condition in a subject are also provided, said methods comprising administering to said subject an agent that modulates the activity or expression of at least one or more of the markers identified by the methods of this invention.

It is to be understood that the embodiments of the present invention which have been described are merely illustrative of some of the applications of the principles of the present invention. Numerous modifications may be made by those skilled in the art based upon the teachings presented herein without departing from the true scope of the invention.

### Examples

### Example 1: A Representative Method for the Separation of Phagocytic Cells from Non-Phagocytic Cells and the Analysis of Expression Profiles

1. With reference to FIG. 1C, coat plates with avidin.
2. Add biotinylated antibody to non-phagocytic blood cell (e.g., T cells) to the wells, incubate for 30 min at RT, wash wells.
3. Add magnetic beads.
4. Add WBC blood sample.
5. Incubate at 37°C (30 minutes - 1 hour).
6. Following phagocytosis of beads by phagocytic cells and binding of avidin-biotin-antibody to non-phagocytic cells, place plate on top of magnet and wash (the phagocytic cells that internalized the magnetic beads and the non-phagocytic cells bound to the antibody will stay; all other cells will be washed away).
7. Remove magnet and collect phagocytic cells.
8. Isolate RNA from phagocytic cells (e.g., cells bound to a magnetic bead) and of non-phagocytic cells (e.g., those cells attached to the bottom of the wells via the anti-non-phagocytic cell biotinylated antibody-avidin bound), prepare cDNA, cRNA and use to differentiate genetic profiles (e.g., whole gene arrays and/or cancer gene arrays) of phagocytic and non-phagocytic cells.
9. Isolate DNA from each cell sample and identify disease-DNA signatures selectively present in phagocytes (i.e., absent in non-phagocytes); compare the profiles (e.g., whole gene arrays, DNA mutations and/or SNPs obtained in phagocytic and non-phagocytic cells).
10. Isolate protein from each cell sample, run Western blots using antibodies to known proteins overexpressed in an individual with a cardiovascular disease or condition, and compare the profiles obtained in phagocytic and non-phagocytic cells. Alternatively, use mass spectroscopy to identify the proteins.
11. Isolate lipids from each cell sample and compare quantity and quality, for example using HPLC.

### Example 2: A Representative Method for the Separation of Phagocytic Cells from Non-Phagocytic Cells and the Analysis of Expression Profiles

1. With reference to FIG. 1C, lyse RBCs in blood sample.
2. Cytospin WBC on glass slides.
3. Fix cells in acetone/methanol (-20°C for 5 minutes).
4. Stain with hematoxylin and eosin stain and anti-T cell antibody.
5. Isolate T cells (non-phagocytic) and macrophages (phagocytic) using laser capture microscopy (LCM).
6. Isolate RNA from phagocytic cells and of non-phagocytic cells, prepare cDNA, cRNA and use to differentiate genetic profiles (e.g., whole gene arrays and/or disease gene arrays) of phagocytic and non-phagocytic cells.
7. Isolate DNA from each cell sample, run DNA arrays, and compare the profiles (e.g., whole gene arrays, DNA mutations and/or SNPs) obtained in phagocytic and non-phagocytic cells.
8. Isolate protein from each cell sample, run Western blots using antibodies to known proteins overexpressed in an individual with a cardiovascular disease or condition, and compare the profiles obtained in phagocytic and non-phagocytic cells. Alternatively, use mass spectroscopy to identify the proteins.
9. Isolate lipids from each cell sample and compare quantity and quality, for example using HPLC.

### Example 3: A Representative Method for the Separation of Phagocytic Cells from Non-Phagocytic Cells and the Analysis of Expression Profiles

1. With reference to FIG. 1C, lyse RBC from a blood sample.
2. Use magnetic antibody-conjugated beads to isolate non-phagocytic (e.g., T cells) and phagocytic cells (e.g., neutrophils and/or macrophages and/or monocytes) from whole blood.
3. Isolate RNA from each cell sample, prepare cDNA, cRNA and use to differentiate genetic profiles (e.g., cancer gene array) of phagocytic and non-phagocytic cells.
4. Isolate DNA from each cell sample, run DNA arrays, and compare the profiles obtained in phagocytic and non-phagocytic cells.
5. Isolate protein from each cell sample, run Western blots using antibodies to known proteins overexpressed in an individual with a cardiovascular disease or condition, and compare the profiles obtained in phagocytic and non-phagocytic cells. Alternatively, use mass spectroscopy to identify the proteins.
6. Isolate lipids from each cell sample and compare quantity and quality, for example using HPLC.

### Example 4: A Representative Method for the Separation of Phagocytic Cells from Non-Phagocytic Cells and the Analysis of Expression Profiles

1. With reference to FIG. 1C, stain WBC with fluorescent antibodies specific against a particular cell subpopulation (e.g., neutrophils, macrophages, monocytes, T cells and the like).
2. Sort the cells (e.g., by FACS).
3. Isolate RNA from each cell sample, prepare cDNA, cRNA and use to differentiate genetic profiles (e.g., gene array) of phagocytic and non-phagocytic cells.
4. Isolate DNA from each cell sample, run DNA arrays, and compare the profiles obtained in phagocytic and non-phagocytic cells.
5. Isolate protein from each cell sample, run Western blots using antibodies to known proteins overexpressed in an individual with a cardiovascular disease or condition, and compare the profiles obtained in phagocytic and non-phagocytic cells. Alternatively, use mass spectroscopy to identify the proteins.
6. Isolate lipids from each cell sample and compare quantity and quality, for example using HPLC.

### Example 5: A Representative Method for the Separation of Phagocytic Cells

### from Non-Phagocytic Cells and the Analysis of Expression Profiles

1. With reference to FIG. 1D, stain WBC with fluorescent antibodies to each cell subpopulation (e.g., neutrophils, macrophages, monocytes, and T cells), and then stain with DNA dye (e.g., propidium iodide).
2. Sort the cells (FACS) into T cells, neutrophils (2n), neutrophils (>2n), macrophages (2n), macrophages (>2n), monocytes (2n), and monocytes (>2n).
3. Isolate RNA from T cells, neutrophils (>2n), macrophages (>2n), and monocytes (>2n). Then prepare cDNA, cRNA and use to differentiate genetic profiles (e.g., disease gene array) of phagocytic and non-phagocytic cells.
4. Isolate DNA from T cells, neutrophils (>2n), macrophages (>2n), and monocytes (>2n). Run DNA arrays and compare the profiles obtained in phagocytic and nonphagocytic cells.
5. Isolate protein from T cells, neutrophils (>2n), macrophages (>2n), and monocytes (>2n). Run Western blots using antibodies to known proteins overexpressed in an individual with a cardiovascular disease or condition, and compare the profiles obtained in phagocytic and non-phagocytic cells. Alternatively, use mass spectroscopy to identify the proteins.
6. Isolate lipids from T cells, neutrophils (>2n), macrophages (>2n), and monocytes (>2n). Compare quantity and quality of lipids, for example using HPLC.

## Claims

1. A method for identifying one or more markers for a cardiovascular disease or condition comprising:
a) preparing a first profile of analytes by detecting said analytes from a subject's phagocytic cells, wherein the subject has said disease or condition;
preparing a second profile of said analytes by detecting said analytes from the subject's non-phagocytic cells;
identifying a first set of differences between the first and second profiles, wherein the first set of differences is specific to the first profile relative to the second profile;
b) preparing a third profile of said analytes by detecting said analytes from a control subject's phagocytic cells, wherein the control subject does not have said disease or condition;
preparing a fourth profile of said analytes by detecting said analytes from the control subject's non-phagocytic cells;
identifying a second set of differences between the third and fourth profiles, wherein the second set of differences is specific to the third profile relative to the fourth profile;
c) identifying one or more analytes specific to the first set of differences relative to the second set of differences, the identified analytes being markers of said disease or condition;
d) obtaining a fifth profile of said analytes from cells or tissues affected by said disease or condition in the subject having said disease or condition;
obtaining a sixth profile of said analytes from cells or tissues not affected by said disease or condition in the subject having said disease or condition;
identifying a third set of differences between the fifth and sixth profiles, wherein the third set of differences is specific to the fifth profile relative to the sixth profile; and
e) identifying at least one of the one or more markers of c) present in the third set of differences.

2. A method for identifying one or more markers of a cardiovascular disease or condition comprising:
a) preparing a first profile of analytes by detecting said analytes from a subject's phagocytic cells, wherein the subject has said disease or condition;
preparing a second profile of said analytes by detecting said analytes from a control subject's phagocytic cells, wherein the control subject does not have said disease or condition;
identifying a first set of differences between the first and second profiles, wherein the first set of differences is specific to the first profile relative to the second profile;
b) preparing a third profile of said analytes by detecting said analytes from the subject's non-phagocytic cells;
preparing a fourth profile of said analytes by detecting said analytes from the control subject's non-phagocytic cells;
identifying a second set of differences between the third and fourth profiles, wherein the second set of differences is specific to the third profile relative to the fourth profile;
c) identifying one or more analytes specific to the first set of differences relative to the second set of differences, the identified analytes being markers of said disease or condition;
d) obtaining a fifth profile of said analytes from cells or tissues affected by said disease or condition in the subject having said disease or condition;
obtaining a sixth profile of said analytes from cells or tissues not affected by said disease or condition in the subject having said disease or condition;
identifying a third set of differences between the fifth and sixth profiles, wherein the third set of differences is specific to the fifth profile relative to the sixth profile; and
e) identifying at least one of the one or more markers of c) present in the third set of differences.

3. A method for identifying one or more markers of a cardiovascular disease or condition comprising:
a) preparing a first profile of analytes by detecting analytes from a subject's phagocytic cells, wherein the subject has said disease or condition;
obtaining a second profile of analytes from a control subject's phagocytic cells, wherein the control subject does not have said disease or condition by data mining;
identifying a first set of differences between the first and second profiles, wherein the first set of differences is specific to the first profile relative to the second profile;
b) preparing a third profile of said analytes by detecting said analytes from the subject's non-phagocytic cells;
obtaining a fourth profile of said analytes from the controls' subject non-phagocytic cells by data mining;
identifying a second set of differences between the third and fourth profiles, wherein the second set of differences is specific to the third profile relative to the fourth profile; and
c) identifying one or more analytes specific to the first set of differences relative to the second set of differences, the identified analytes being markers of said disease or condition;
d) obtaining a fifth profile of said analytes from cells or tissues affected by said disease or condition by data mining;
obtaining a sixth profile of said analytes from cells or tissues not affected by said disease or condition by data mining;
identifying a third set of differences between the fifth and sixth profiles, wherein the third set of differences is specific to the fifth profile relative to the sixth profile; and
e) identifying at least one of the one or more markers of c) present in the third set of differences.

4. A method for identifying one or more markers of a cardiovascular disease or condition comprising:
a) preparing a first profile of analytes by detecting said analytes from a subject's phagocytic cells, wherein the subject has said disease or condition;
preparing a second profile of said analytes by detecting said analytes from the subject's non-phagocytic cells;
identifying a first set of differences between the first and second profiles, wherein the first set of differences is specific to the first profile relative to the second profile;
b) preparing a third profile of said analytes by detecting said analytes from the subject's cells or tissues affected by said disease or condition;
preparing a fourth profile of said analytes by detecting said analytes from the subject's cells or tissues not affected by said disease or condition;
identifying a second set of differences between the third and fourth profiles, wherein the second set of differences is specific to the third profile relative to the fourth profile;
c) identifying one or more analytes present in both the first set of differences and the second set of differences, the identified analytes being markers of said disease or condition;
d) preparing a fifth profile of said analytes by detecting said analytes from a control subject's phagocytic cells, wherein the control subject does not have said disease or condition;
identifying a third set of differences between the first and fifth profiles, wherein the third set of differences is specific to the first profile relative to the fifth profile;
e) identifying at least one of the one or more markers of c) present in the third set of differences.

5. A method for identifying one or more markers of a cardiovascular disease or condition comprising:
a) preparing a first profile of analytes by detecting said analytes from a subject's >2n phagocytic cells, wherein the subject has said disease or condition;
preparing a second profile of said analytes by detecting said analytes from the subject's =2n phagocytic cells;
identifying a first set of differences between the first and second profiles, wherein the first set of differences is specific to the first profile relative to the second profile;
b) preparing a third profile of said analytes by detecting said analytes from a control subject's >2n phagocytic cells, wherein the control subject does not have said disease or condition;
preparing a fourth profile of said analytes by detecting said analytes from the control subject's =2n phagocytic cells;
identifying a second set of differences between the third and fourth profiles, wherein the second set of differences is specific to the third profile relative to the fourth profile; and
c) identifying one or more analytes specific to the first set of differences relative to the second set of differences, the identified analytes being markers of said disease or condition;
d) obtaining a fifth profile of said analytes from cells or tissues affected by said disease or condition in the subject having said disease or condition;
obtaining a sixth profile of said analytes from cells or tissues not affected by said disease or condition in the subject having said disease or condition;
identifying a third set of differences between the fifth and sixth profiles, wherein the third set of differences is specific to the fifth profile relative to the sixth profile; and
e) identifying at least one of the one or more markers of c) present in the third set of differences.

6. The method of any one of claims 1 to 5, wherein the analytes are nucleic acids, proteins, lipids, carbohydrates, metabolites, or combinations thereof;
optionally wherein the nucleic acids are nucleotides, oligonucleotides, DNAs, RNAs, or DNA-RNA hybrids;
optionally wherein the proteins are amino acids, peptides, enzymes, antigens, antibodies, cytokines, lipoproteins, glycoproteins, or hormones;
optionally wherein the lipids are fatty acids, neutral fats, phosphatides, cholesterol, cholesterol esters, triglycerides, glycolipids, glycerolipids, glycerophospholipids, sphingolipids, sterol lipids, prenol lipids, saccharolipids, polyketides, choline glycerophospholipid, ethanolamine glycerophospholipid, phosphatidylinositol, phosphatidylglycerol, phosphatidylserine, lyso-choline glycerophospholipid, lyso-ethanolamine glycerophospholipid, phosphatidic acid, lyso-phosphatidic acid, sphingomyelin, galactosylceramide, glucosylceramide, free fatty acids, prostaglandins, triacylglycerol, diacylglycerol, monoacylglycerol, acyl-CoA, acylcarnitine, oxysterol, ceramide, cardiolipin, sphingoid base-1-phosphate, shingosine, lyso-sphingomyelin, gangliosides, plasmalogen, sulfatide, low density lipoproteins (LDLs), very low density lipoproteins (VLDLs), high density lipoproteins (HDLs), sphingoid base-1-phosphates or derivatives thereof;
optionally wherein the carbohydrates are monosaccharides, disaccharides, polysaccharides, oligosaccharides, or derivatives thereof; and
optionally wherein the metabolites are primary metabolites, secondary metabolites, organic metabolites, inorganic metabolites, prostaglandins, hydroxyeicosatetraenoic acids, hydroxyoctadecadienoic acids, steroids, bile acids, vitamins, or derivatives thereof.

7. The method of any one of claims 1 to 6, wherein the profile is a nucleic acid profile, a protein profile, a lipid profile, a carbohydrate profile, a metabolite profile, or a combination thereof;
optionally wherein the nucleic acid profile is a genotypic profile, a single nucleotide polymorphism profile, a gene mutation profile, a gene copy number profile, a DNA methylation profile, a DNA acetylation profile, a chromosome dosage profile, a gene expression profile, or a combination thereof; and
optionally wherein the protein profile is a protein expression profile, a protein activation profile, or a combination thereof; and further optionally wherein the protein activation profile comprises determining a phosphorylation state, an ubiquitination state, a myristoylation state, or a conformational state of the one or more markers.

8. The method of any one of claims 1 to 7, wherein the profile is detected by a qualitative assay, a quantitative assay, or a combination thereof;
optionally wherein the quantitative assay uses sequencing, direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), polymerase chain reaction (PCR) analysis, quantitative PCR, real-time PCR, fluorescence assay, colorimetric assay, chemiluminescent assay, or a combination thereof.

9. The method of any one of claims 1 to 8, wherein the profile is a nucleic acid profile detected by polymerase chain reaction (PCR) analysis, sequencing analysis, electrophoretic analysis, restriction fragment length polymorphism (RFLP) analysis, Northern blot analysis, reverse-transcriptase-PCR analysis (RT-PCR), quantitative PCR, quantitative RT-PCR, allele-specific oligonucleotide hybridization analysis, comparative genomic hybridization, heteroduplex mobility assay (HMA), single strand conformational polymorphism (SSCP), denaturing gradient gel electrophisis (DGGE), RNAase mismatch analysis, mass spectrometry, mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), Southern blot analysis, in situ hybridization, fluorescence in situ hybridization (FISH), chromogenic in situ hybridization (CISH), immunohistochemistry (IHC), microarray, comparative genomic hybridization, karyotyping, multiplex ligation-dependent probe amplification (MLPA), Quantitative Multiplex PCR of Short Fluorescent Fragments (QMPSF), microscopy, methylation specific PCR (MSP) assay, HpaII tiny fragment Enrichment by Ligation-mediated PCR (HELP) assay, radioactive acetate labeling assays, colorimetric DNA acetylation assay, chromatin immunoprecipitation combined with microarray (ChIP-on-chip) assay, restriction landmark genomic scanning, Methylated DNA immunoprecipitation (MeDIP), molecular break light assay for DNA adenine methyltransferase activity, chromatographic separation, methylation-sensitive restriction enzyme analysis, surface plasmon resonance, bisulfite-driven conversion of non-methylated cytosine to uracil, methyl-binding PCR analysis, or a combination thereof.
optionally wherein the sequencing analysis is selected from the group consisting of direct sequencing, random shotgun sequencing, Sanger dideoxy termination sequencing, whole-genome sequencing, sequencing by hybridization, pyrosequencing, capillary electrophoresis, gel electrophoresis, duplex sequencing, cycle sequencing, single-base extension sequencing, solid-phase sequencing, high-throughput sequencing, massively parallel signature sequencing, emulsion PCR, sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD® sequencing, MS-PET sequencing, mass spectrometry, and a combination thereof;
or wherein the profile is a protein profile detected by an immunohistochemistry assay, an enzyme-linked immunosorbent assay (ELISA), chromatography, liquid chromatography, size exclusion chromatography, high performance liquid chromatography (HPLC), gas chromatography, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, surface plasmon resonance, microfluidic chip-based assays, Western blotting assay, or a combination thereof;
or wherein the profile is a lipid profile detected by chromatography, liquid chromatography, size exclusion chromatography, high performance liquid chromatography (HPLC), gas chromatography, mass spectrometry, matrix assisted laser desorption/ionization-time of flight (MALDI-TOF) mass spectrometry, tandem mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assays, detection of fluorescence, detection of chemiluminescence, or a combination thereof;
or wherein the profile is a carbohydrate profile detected by chromatography, liquid chromatography, size exclusion chromatography, high performance anion exchange chromatography with pulsed amperometric detection (HPAEC-PAD), liquid chromatography, gas chromatography, fluorescent assay, mass spectrometry, tandem mass spectrometry, matrix assisted laser desorption/ionization-time of flight (MALDI-TOF) mass spectrometry, electrospray ionization (ESI) mass spectrometry, surface-enhanced laser deorption/ionization-time of flight (SELDI-TOF) mass spectrometry, quadrupole-time of flight (Q-TOF) mass spectrometry, atmospheric pressure photoionization mass spectrometry (APPI-MS), Fourier transform mass spectrometry (FTMS), matrix-assisted laser desorption/ionization-Fourier transform-ion cyclotron resonance (MALDI-FT-ICR) mass spectrometry, secondary ion mass spectrometry (SIMS), radioimmunoassays, microfluidic chip-based assays, detection of fluorescence, detection of chemiluminescence, or a combination thereof.

10. The method of any one of claims 1 to 9, wherein the cardiovascular disease or condition is selected from the group consisting of myocardial infarction, coronary artery disease, percutaneous transluminal coronary angioplasty (PTCA), coronary artery bypass surgery (CABG), restenosis, peripheral arterial disease, stroke, abdominal aorta aneurysm, intracranial aneurysm, large artery atherosclerotic stroke, cardiogenic stroke, an early onset myocardial infarction, heart failure, pulmonary embolism, acute coronary syndrome (ACS), angina, cardiac hypertrophy, arteriosclerosis, myocarditis, pancarditis, endocarditis, hypertension, congestive heart failure, atherosclerosis, cerebrovascular disease, declining cardiac health, ischemic heart disease, pericarditis, cardiogenic shock, alcoholic cardiomyopathy, congenital heart disease, ischemic cardiomyopathy, hypertensive cardiomyopathy, valvular cardiomyopathy, inflammatory cardiomyopathy, cardiomyopathy secondary to a systemic metabolic disease, dilated cardiomyopathy, hypertrophic cardiomyopathy, arrhythmogenic right ventricular cardiomyopathy, restrictive cardiomyopathy, noncompaction cardiomyopathy, valvular heart disease, hypertensive heart disease, myocardial ischemic attack, unstable angina, myocardial rupture, cardiogenic shock, embolism, deep vein thrombosis, arrhythmia, arrhythmogenic right ventricular cardiomyopathy, diabetic cardiomyopathy, mitral regurgitation, mitral valve prolapse, peripheral vascular disease, artery disease, carotid artery disease, deep vein thrombosis, venous diseases, cerebrovascular disease, arterial aneurysm, left ventricular hypertrophy, hypertensive renal disease, hypertensive retinal disease, vasculitis, left main disease, arterial vascular disease, venous vascular disease, thrombosis of the microcirculation, a transient cerebrovascular accident, limb ischemia, aneurysm, thrombosis, superficial venous thrombosis, and deep venous thrombosis.

11. The method of any one of claims 1 to 4, further comprising extracting cellular contents from the phagocytic cells and the non-phagocytic cells before a); optionally wherein the cellular contents of the phagocytic cells comprise viable diseased cells, dead diseased cells, apoptotic diseased cells, circulating tumor cells, infectious agents, fetal cells, trophoblasts, or fragments thereof.

12. The method of any one of claims 1 to 5, wherein the phagocytic cells are professional phagocytic cells, non-professional phagocytic cells, or mixtures thereof, or wherein the non-phagocytic cells are T cells, B cells, null cells, basophils, or mixtures thereof;
optionally wherein the professional phagocytic cells are neutrophils, macrophages, monocytes, dendritic cells, foam cells, mast cells, eosinophils, or mixtures thereof;
optionally wherein the non-professional phagocytic cells are epithelial cells, endothelial cells, fibroblasts, mesenchymal cells, or mixtures thereof; and
optionally wherein the phagocytic cells or the non-phagocytic cells are isolated from a bodily fluid sample, tissues, or cells of the subject; optionally wherein the bodily fluid sample is blood, urine, stool, saliva, lymph fluid, cerebrospinal fluid, synovial fluid, cystic fluid, ascites, pleural effusion, fluid obtained from a pregnant woman in the first trimester, fluid obtained from a pregnant woman in the second trimester, fluid obtained from a pregnant woman in the third trimester, maternal blood, amniotic fluid, chorionic villus sample, maternal urine, maternal saliva, placental sample, fetal blood, lavage and cervical vaginal fluid, interstitial fluid, or ocular fluid.

## Patentansprüche

1. Verfahren zur Identifizierung eines oder mehrerer Marker für eine kardiovaskuläre Krankheit oder ein kardiovaskuläres Leiden, umfassend:
a) Erstellen eines ersten Profils von Analyten durch Nachweisen der Analyten aus phagozytischen Zellen eines Subjekts, wobei das Subjekt die Krankheit oder das Leiden hat;
Erstellen eines zweiten Profils der Analyten durch Nachweisen der Analyten aus nicht-phagozytischen Zellen des Subjekts;
Identifizieren eines ersten Satzes von Differenzen zwischen den ersten und zweiten Profilen, wobei der erste Satz von Differenzen für das erste Profil in Bezug auf das zweite Profil spezifisch ist;
b) Erstellen eines dritten Profils der Analyten durch Nachweisen der Analyten aus phagozytischen Zellen eines Kontrollsubjekts, wobei das Kontrollsubjekt die Krankheit oder das Leiden nicht hat;
Erstellen eines vierten Profils der Analyten durch Nachweisen der Analyten aus nicht-phagozytischen Zellen des Kontrollsubjekts;
Identifizieren eines zweiten Satzes von Differenzen zwischen den dritten und vierten Profilen, wobei der zweite Satz von Differenzen für das dritte Profil in Bezug auf das vierte Profil spezifisch ist;
c) Identifizieren eines oder mehrerer Analyten, die für den ersten Satz von Differenzen in Bezug auf den zweiten Satz von Differenzen spezifisch sind, wobei die identifizierten Analyten Marker der Krankheit oder des Leidens sind;
d) Erhalten eines fünften Profils der Analyten aus von der Krankheit oder dem Leiden betroffenen Zellen oder Geweben im Subjekt mit der Krankheit oder dem Leiden;
Erhalten eines sechsten Profils der Analyten aus von der Krankheit oder dem Leiden nicht betroffenen Zellen oder Geweben im Subjekt mit der Krankheit oder dem Leiden;
Identifizieren eines dritten Satzes von Differenzen zwischen den fünften und sechsten Profilen, wobei der dritte Satz von Differenzen für das fünfte Profil in Bezug auf das sechste Profil spezifisch ist; und
e) Identifizieren mindestens eines des einen oder der mehreren Marker von c), die im dritten Satz von Differenzen vorhanden sind.

2. Verfahren zur Identifizierung eines oder mehrerer Marker einer kardiovaskulären Krankheit oder eines kardiovaskulären Leidens, umfassend:
a) Erstellen eines ersten Profils von Analyten durch Nachweisen der Analyten aus phagozytischen Zellen eines Subjekts, wobei das Subjekt die Krankheit oder das Leiden hat;
Erstellen eines zweiten Profils der Analyten durch Nachweisen der Analyten aus phagozytischen Zellen eines Kontrollsubjekts, wobei das Kontrollsubjekt die Krankheit oder das Leiden nicht hat;
Identifizieren eines ersten Satzes von Differenzen zwischen den ersten und zweiten Profilen, wobei der erste Satz von Differenzen für das erste Profil in Bezug auf das zweite Profil spezifisch ist;
b) Erstellen eines dritten Profils der Analyten durch Nachweisen der Analyten aus nicht-phagozytischen Zellen des Subjekts;
Erstellen eines vierten Profils der Analyten durch Nachweisen der Analyten aus nicht-phagozytischen Zellen des Kontrollsubjekts;
Identifizieren eines zweiten Satzes von Differenzen zwischen den dritten und vierten Profilen, wobei der zweite Satz von Differenzen für das dritte Profil in Bezug auf das vierte Profil spezifisch ist;
c) Identifizieren eines oder mehrerer Analyten, die für den ersten Satz von Differenzen in Bezug auf den zweiten Satz von Differenzen spezifisch sind, wobei die identifizierten Analyten Marker der Krankheit oder des Leidens sind;
d) Erhalten eines fünften Profils der Analyten aus von der Krankheit oder dem Leiden betroffenen Zellen oder Geweben im Subjekt mit der Krankheit oder dem Leiden;
Erhalten eines sechsten Profils der Analyten aus von der Krankheit oder dem Leiden nicht betroffenen Zellen oder Geweben im Subjekt mit der Krankheit oder dem Leiden;
Identifizieren eines dritten Satzes von Differenzen zwischen den fünften und sechsten Profilen, wobei der dritte Satz von Differenzen für das fünfte Profil in Bezug auf das sechste Profil spezifisch ist; und
e) Identifizieren mindestens eines des einen oder der mehreren Marker von c), die im dritten Satz von Differenzen vorhanden sind.

3. Verfahren zur Identifizierung eines oder mehrerer Marker einer kardiovaskulären Krankheit oder eines kardiovaskulären Leidens, umfassend:
a) Erstellen eines ersten Profils von Analyten durch Nachweisen der Analyten aus phagozytischen Zellen eines Subjekts, wobei das Subjekt die Krankheit oder das Leiden hat;
Erhalten eines zweiten Profils von Analyten aus phagozytischen Zellen eines Kontrollsubjekts durch Datenauswertung, wobei das Kontrollsubjekt die Krankheit oder das Leiden nicht hat;
Identifizieren eines ersten Satzes von Differenzen zwischen den ersten und zweiten Profilen, wobei der erste Satz von Differenzen für das erste Profil in Bezug auf das zweite Profil spezifisch ist;
b) Erstellen eines dritten Profils der Analyten durch Nachweisen der Analyten aus nicht-phagozytischen Zellen des Subjekts;
Erhalten eines vierten Profils der Analyten aus nicht-phagozytischen Zellen des Kontrollsubjekts durch Datenauswertung;
Identifizieren eines zweiten Satzes von Differenzen zwischen den dritten und vierten Profilen, wobei der zweite Satz von Differenzen für das dritte Profil in Bezug auf das vierte Profil spezifisch ist; und
c) Identifizieren eines oder mehrerer Analyten, die für den ersten Satz von Differenzen in Bezug auf den zweiten Satz von Differenzen spezifisch sind, wobei die identifizierten Analyten Marker der Krankheit oder des Leidens sind;
d) Erhalten eines fünften Profils der Analyten aus von der Krankheit oder dem Leiden betroffenen Zellen oder Geweben durch Datenauswertung;
Erhalten eines sechsten Profils der Analyten aus von der Krankheit oder dem Leiden nicht betroffenen Zellen oder Geweben durch Datenauswertung;
Identifizieren eines dritten Satzes von Differenzen zwischen den fünften und sechsten Profilen, wobei der dritte Satz von Differenzen für das fünfte Profil in Bezug auf das sechste Profil spezifisch ist; und
e) Identifizieren mindestens eines des einen oder der mehreren Marker von c), die im dritten Satz von Differenzen vorhanden sind.

4. Verfahren zur Identifizierung eines oder mehrerer Marker einer kardiovaskulären Krankheit oder eines kardiovaskulären Leidens, umfassend:
a) Erstellen eines ersten Profils von Analyten durch Nachweisen der Analyten aus phagozytischen Zellen eines Subjekts, wobei das Subjekt die Krankheit oder das Leiden hat;
Erstellen eines zweiten Profils der Analyten durch Nachweisen der Analyten aus nicht-phagozytischen Zellen des Subjekts;
Identifizieren eines ersten Satzes von Differenzen zwischen den ersten und zweiten Profilen, wobei der erste Satz von Differenzen für das erste Profil in Bezug auf das zweite Profil spezifisch ist;
b) Erstellen eines dritten Profils der Analyten durch Nachweisen der Analyten aus von der Krankheit oder dem Leiden betroffenen Zellen oder Geweben des Subjekts;
Erstellen eines vierten Profils der Analyten durch Nachweisen der Analyten aus von der Krankheit oder dem Leiden nicht betroffenen Zellen oder Geweben des Subjekts;
Identifizieren eines zweiten Satzes von Differenzen zwischen den dritten und vierten Profilen, wobei der zweite Satz von Differenzen für das dritte Profil in Bezug auf das vierte Profil spezifisch ist;
c) Identifizieren eines oder mehrerer Analyten, die sowohl im ersten Satz von Differenzen als auch im zweiten Satz von Differenzen vorhanden sind, wobei die identifizierten Analyten Marker der Krankheit oder des Leidens sind;
d) Erstellen eines fünften Profils der Analyten durch Nachweisen der Analyten aus phagozytischen Zellen eines Kontrollsubjekts, wobei das Kontrollsubjekt die Krankheit oder das Leiden nicht hat;
Identifizieren eines dritten Satzes von Differenzen zwischen den ersten und fünften Profilen, wobei der dritte Satz von Differenzen für das erste Profil in Bezug auf das fünfte Profil spezifisch ist;
e) Identifizieren mindestens eines des einen oder der mehreren Marker von c), die im dritten Satz von Differenzen vorhanden sind.

5. Verfahren zur Identifizierung eines oder mehrerer Marker einer kardiovaskulären Krankheit oder eines kardiovaskulären Leidens, umfassend:
a) Erstellen eines ersten Profils von Analyten durch Nachweisen der Analyten aus > 2n phagozytischen Zellen eines Subjekts, wobei das Subjekt die Krankheit oder das Leiden hat;
Erstellen eines zweiten Profils der Analyten durch Nachweisen der Analyten aus = 2n phagozytischen Zellen des Subjekts;
Identifizieren eines ersten Satzes von Differenzen zwischen den ersten und zweiten Profilen, wobei der erste Satz von Differenzen für das erste Profil in Bezug auf das zweite Profil spezifisch ist;
b) Erstellen eines dritten Profils der Analyten durch Nachweisen der Analyten aus > 2n phagozytischen Zellen eines Kontrollsubjekts, wobei das Kontrollsubjekt die Krankheit oder das Leiden nicht hat;
Erstellen eines vierten Profils der Analyten durch Nachweisen der Analyten aus = 2n phagozytischen Zellen des Kontrollsubjekts;
Identifizieren eines zweiten Satzes von Differenzen zwischen den dritten und vierten Profilen, wobei der zweite Satz von Differenzen für das dritte Profil in Bezug auf das vierte Profil spezifisch ist; und
c) Identifizieren eines oder mehrerer Analyten, die für den ersten Satz von Differenzen in Bezug auf den zweiten Satz von Differenzen spezifisch sind, wobei die identifizierten Analyten Marker der Krankheit oder des Leidens sind;
d) Erhalten eines fünften Profils der Analyten aus von der Krankheit oder dem Leiden betroffenen Zellen oder Geweben im Subjekt mit der Krankheit oder dem Leiden;
Erhalten eines sechsten Profils der Analyten aus von der Krankheit oder dem Leiden nicht betroffenen Zellen oder Geweben im Subjekt mit der Krankheit oder dem Leiden;
Identifizieren eines dritten Satzes von Differenzen zwischen den fünften und sechsten Profilen, wobei der dritte Satz von Differenzen für das fünfte Profil in Bezug auf das sechste Profil spezifisch ist; und
e) Identifizieren mindestens eines des einen oder der mehreren Marker von c), die im dritten Satz von Differenzen vorhanden sind.

6. Verfahren nach einem der Ansprüche 1 bis 5, wobei es sich bei den Analyten um Nucleinsäuren, Proteine, Lipide, Kohlenhydrate, Metaboliten oder Kombinationen davon handelt;
wobei die Nucleinsäuren gegebenenfalls Nucleotide, Oligonucleotide, DNAs, RNAs oder DNA-RNA-Hybride sind;
wobei die Proteine gegebenenfalls Aminosäuren, Peptide, Enzyme, Antigene, Antikörper, Cytokine, Lipoproteine, Glycoproteine oder Hormone sind;
wobei die Lipide gegebenenfalls Fettsäuren, neutrale Fette, Phosphatide, Cholesterol, Cholesterolester, Triglyceride, Glycolipide, Glycerolipide, Glycerophospholipide, Sphingolipide, Sterol-Lipide, Prenol-Lipide, Saccharolipide, Polyketide, Cholinglycerophospholipid, Ethanolaminglycerophospholipid, Phosphatidylinositol, Phosphatidylglycerol, Phosphatidylserin, Lyso-Cholinglycerophospholipid, Lyso-Ethanolaminglycerophospholipid, Phosphatidsäure, Lyso-Phosphatidsäure, Sphingomyelin, Galactosylceramid, Glucosylceramid, freie Fettsäuren, Prostaglandine, Triacylglycerol, Diacylglycerol, Monoacylglycerol, Acyl-CoA, Acylcarnitin, Oxysterol, Ceramid, Cardiolipin, Sphingoidbase-1-Phosphat, Sphingosin, Lyso-Sphingomyelin, Ganglioside, Plasmalogen, Sulfatide, Lipoproteine mit niedriger Dichte (LDLs), Lipoproteine mit sehr niedriger Dichte (VLDLs), Lipoproteine mit hoher Dichte (HDLs), Sphingoidbase-1-Phosphate oder Derivate davon sind;
wobei die Kohlenhydrate gegebenenfalls Monosaccharide, Disaccharide, Polysaccharide, Oligosaccharide oder Derivative davon sind; und
wobei die Metaboliten gegebenenfalls primäre Metaboliten, sekundäre Metaboliten, organische Metaboliten, anorganische Metaboliten, Prostaglandine, Hydroxyeicosatetraensäuren, Hydroxyoctadecadiensäuren, Steroide, Gallensäuren, Vitamine oder Derivate davon sind.

7. Verfahren nach einem der Ansprüche 1 bis 6, wobei das Profil ein Nucleinsäureprofil, ein Proteinprofil, ein Lipidprofil, ein Kohlenhydratprofil, ein Metabolitprofil oder eine Kombination davon ist;
wobei das Nucleinsäureprofil gegebenenfalls ein genotypisches Profil, ein Einzelnucleotid-Polymorphismusprofil, ein Genmutationsprofil, ein Genkopienummernprofil, ein DNA-Methylierungsprofil, ein DNA-Acetylierungsprofil, ein Chromosomendosierungsprofil, ein Genexpressionsprofil oder eine Kombination davon ist; und
wobei das Proteinprofil gegebenenfalls ein Proteinexpressionsprofil, ein Proteinaktivierungsprofil oder eine Kombination davon ist; und wobei das Proteinaktivierungsprofil ferner gegebenenfalls ein Bestimmen eines Phosphorylierungszustands, eines Ubiquitinierungszustands, eines Myristoylierungszustands oder eines Konformationszustands des einen oder der mehreren Marker umfasst.

8. Verfahren nach einem der Ansprüche 1 bis 7, wobei das Profil durch einen qualitativen Assay, einen quantitativen Assay oder eine Kombination davon nachgewiesen wird;
wobei der quantitative Assay gegebenenfalls Sequenzierung, direkte Sequenzierung, Schrotschuss-Zufallssequenzierung, Didesoxy-Terminationssequenzierung nach Sanger, Gesamtgenomsequenzierung, Sequenzierung durch Hybridisierung, Pyrosequenzierung, Kapillarelektrophorese, Gelelektrophorese, Duplex-Sequenzierung, Zyklus-Sequenzierung, Einzelbasenerweiterungs-Sequenzierung, Festphasensequenzierung, Hochdurchsatz-Sequenzierung, massiv parallele Signatursequenzierung, Emulsions-PCR, Sequenzierung durch reversiblen Farbstoffterminator, Paired-End-Sequenzierung, kurzfristige Sequenzierung, Exonucleasen-Sequenzierung, Sequenzierung durch Ligation, Kurz-Lese-Sequenzierung, Einzelmolekül-Sequenzierung, Sequenzierung durch Synthese, Echtzeit-Sequenzierung, Umkehrterminator-Sequenzierung, Nanoporen-Sequenzierung, 454-Sequenzierung, Sequenzierung durch Solexa Genome Analyzer, SOLiD® Sequenzierung, MS-PET-Sequenzierung, Massenspektrometrie, matrixgestützte Laser-Desorption/Ionisations-Flugzeit (MALDI-TOF)-Massenspektrometrie, Elektrospray-Ionisations (ESI)-Massenspektrometrie, oberflächenerweiterte Laser-Desorption/Ionisations-Flugzeit (SELDI-TOF)-Massenspektrometrie, Quadrupol-Flugzeit (Q-TOF)-Massenspektrometrie, Atmosphärendruck-Photoionisations-Massenspektrometrie (APPI-MS), Fourier-Transformations-Massenspektrometrie (FTMS), matrixgestützte Laser-Desorption/Ionisations-Fourier-Transformations-Ionenzyklotronresonanz (MALDI-FT-ICR)-Massenspektrometrie, Sekundärionen-Massenspektrometrie (SIMS), Polymerase-Kettenreaktions (PCR)-Analyse, quantitative PCR, Echtzeit-PCR, einen Fluoreszenz-Assay, einen kolorimetrischen Assay, einen Chemilumineszenz-Assay oder eine Kombination davon verwendet.

9. Verfahren nach einem der Ansprüche 1 bis 8,
wobei das Profil ein Nucleinsäureprofil ist, das durch Polymerase-Kettenreaktions (PCR)-Analyse, Sequenzierungsanalyse, elektrophoretische Analyse, Restriktionsfragmentlängenpolymorphismus (RFLP)-Analyse, Northern-Blot-Analyse, Umkehrtranskriptase-PCR-Analyse (RT-PCR), quantitative PCR, quantitative RT-PCR, allelspezifische Oligonucleotid-Hybridisierungsanalyse, komparative Genomhybridierung, Heteroduplex-Mobilitätsassay (HMA), Einzelstrang-Konformationspolymorphismus (SSCP), denaturierende Gradientengel-Elektrophorese (DGGE), RNAase-Fehlanpassungsanalyse, Massenspektrometrie, Massenspektrometrie, matrixgestützte Laser-Desorption/Ionisations-Flugzeit (MALDI-TOF)-Massenspektrometrie, Elektrospray-Ionisations (ESI)-Massenspektrometrie, oberflächenerweiterte Laser-Desorption/Ionisations-Flugzeit (SELDI-TOF)-Massenspektrometrie, Quadrupol-Flugzeit (Q-TOF)-Massenspektrometrie, Atmosphärendruck-Photoionisations-Massenspektrometrie (APPI-MS), Fourier-Transformations-Massenspektrometrie (FTMS), matrixgestützte Laser-Desorption/Ionisations-Fourier-Transformations-Ionenzyklotronresonanz (MALDI-FT-ICR)-Massenspektrometrie, Sekundärionen-Massenspektrometrie (SIMS), Southern-Blot-Analyse, In-situ-Hybridisierung, Fluoreszenz-in-situ-Hybridisierung (FISH), chromogene In-situ-Hybridisierung (CISH), Immunhistochemie (IHC), Mikroarray, komparative Genomhybridisierung, Karyotypisierung, multiplexe ligationsabhängige Sondenamplifikation (MLPA), quantitative multiplexe PCR kurzer fluoreszierender Fragmente (QMPSF), Mikroskopie, MSP (methylierungsspezifische PCR)-Assay, HELP (Anreicherung mit winzigen HpaII-Fragmenten durch ligationsvermittelte PCR)-Assay, radioaktive Acetatmarkierungsassays, kolorimetrischen DNA-Acetylierungsassay, Chromatin-Immunpräzipitation kombiniert mit Mikroarray (ChIP-on-chip)-Assay, Restriktionslandmarken-Genom-Scanning, Immunpräzipitation methylierter DNA (MeDIP), molekularen Break-Light-Assay hinsichtlich DNA-Adenin-Methyltransferase-Aktivität, chromatographische Trennung, Analyse methylierungssensitiver Restriktionsenzyme, Oberflächenplasmonresonanz, bisulfitgesteuerte Konversion nicht-methylierten Cytosins in Uracil, Analyse mit methylbindender PCR oder eine Kombination davon nachgewiesen wird;
wobei die Sequenzierungsanalyse gegebenenfalls aus der Gruppe bestehend aus direkter Sequenzierung, Schrotschuss-Zufallssequenzierung, Didesoxy-Terminationssequenzierung nach Sanger, Gesamtgenomsequenzierung, Sequenzierung durch Hybridisierung, Pyrosequenzierung, Kapillarelektrophorese, Gelelektrophorese, Duplex-Sequenzierung, Zyklus-Sequenzierung, Einzelbasenerweiterungs-Sequenzierung, Festphasensequenzierung, Hochdurchsatz-Sequenzierung, massiv paralleler Signatursequenzierung, Emulsions-PCR, Sequenzierung durch reversiblen Farbstoffterminator, Paired-End-Sequenzierung, kurzfristiger Sequenzierung, Exonucleasen-Sequenzierung, Sequenzierung durch Ligation, Kurz-Lese-Sequenzierung, Einzelmolekül-Sequenzierung, Sequenzierung durch Synthese, Echtzeit-Sequenzierung, Umkehrterminator-Sequenzierung, Nanoporen-Sequenzierung, 454-Sequenzierung, Sequenzierung durch Solexa Genome Analyzer, SOLiD® Sequenzierung, MS-PET-Sequenzierung, Massenspektrometrie und einer Kombination davon ausgewählt wird;
oder wobei das Profil ein Proteinprofil ist, das durch einen immunhistochemischen Assay, einen Enzymimmunassay (ELISA), Chromatographie, Flüssigkeitschromatographie, Größenausschlusschromatographie, Hochleistungs-Flüssigkeitschromatographie (HPLC), Gaschromatographie, Massenspektrometrie, Tandem-Massenspektrometrie, matrixgestützte Laser-Desorption/Ionisations-Flugzeit (MALDI-TOF)-Massenspektrometrie, Elektrospray-Ionisations (ESI)-Massenspektrometrie, oberflächenerweiterte Laser-Desorption/Ionisations-Flugzeit (SELDI-TOF)-Massenspektrometrie, Quadrupol-Flugzeit (Q-TOF)-Massenspektrometrie, Atmosphärendruck-Photoionisations-Massenspektrometrie (APPI-MS), Fourier-Transformations-Massenspektrometrie(FTMS), matrixgestützte Laser-Desorption/Ionisations-Fourier-Transformations-Ionenzyklotronresonanz (MALDI-FT-ICR)-Massenspektrometrie, Sekundärionen-Massenspektrometrie (SIMS), Radioimmunoassays, Oberflächenplasmonresonanz, chipgestützte Mikrofluidik-Assays, Western-Blot-Assay, oder eine Kombination davon nachgewiesen wird;
oder wobei das Profil ein Lipidprofil ist, das durch Chromatographie, Flüssigkeitschromatographie, Größenausschlusschromatographie, Hochleistungs-Flüssigkeitschromatographie (HPLC), Gaschromatographie, Massenspektrometrie, matrixgestützte Laser-Desorption/Ionisations-Flugzeit (MALDI-TOF)-Massenspektrometrie, Tandem-Massenspektrometrie, Elektrospray-Ionisations (ESI)-Massenspektrometrie, oberflächenerweiterte Laser-Desorption/Ionisations-Flugzeit (SELDI-TOF)-Massenspektrometrie, Quadrupol-Flugzeit (Q-TOF)-Massenspektrometrie, Atmosphärendruck-Photoionisations-Massenspektrometrie (APPI-MS), Fourier-Transformations-Massenspektrometrie (FTMS), matrixgestützte Laser-Desorption/Ionisations-Fourier-Transformations-Ionenzyklotronresonanz (MALDI-FT-ICR)-Massenspektrometrie, Sekundärionen-Massenspektrometrie (SIMS), Radioimmunoassays, chipgestützte Mikrofluidik-Assays, Fluoreszenznachweis, Chemilumineszenznachweis oder eine Kombination davon nachgewiesen wird;
oder wobei das Profil ein Kohlenhydratprofil ist, das durch Chromatographie, Flüssigkeitschromatographie, Größenausschlusschromatographie, Hochleistungs-Anionenaustauschchromatographie mit Nachweis durch gepulste Amperometrie (HPAEC-PAD), Flüssigkeitschromatographie, Gaschromatographie, Fluoreszenzassay, Massenspektrometrie, Tandem-Massenspektrometrie, matrixgestützte Laser-Desorption/Ionisations-Flugzeit (MALDI-TOF)-Massenspektrometrie, Elektrospray-Ionisations (ESI)-Massenspektrometrie, oberflächenerweiterte Laser-Desorption/Ionisations-Flugzeit (SELDI-TOF)-Massenspektrometrie, Quadrupol-Flugzeit (Q-TOF)-Massenspektrometrie, Atmosphärendruck-Photoionisations-Massenspektrometrie (APPI-MS), Fourier-Transformations-Massenspektrometrie (FTMS), matrixgestützte Laser-Desorption/Ionisations-Fourier-Transformations-Ionenzyklotronresonanz (MALDI-FT-ICR)-Massenspektrometrie, Sekundärionen-Massenspektrometrie (SIMS), Radioimmunoassays, chipgestützte Mikrofluidik-Assays, Fluoreszenznachweis, Chemilumineszenznachweis oder eine Kombination davon nachgewiesen wird.

10. Verfahren nach einem der Ansprüche 1 bis 9,
wobei die kardiovaskuläre Krankheit oder das kardiovaskuläre Leiden aus der Gruppe bestehend aus Myokardinfarkt, koronarer Herzerkrankung, perkutaner transluminaler Koronarangioplastie (PTCA), Koronararterien-Bypass-Chirurgie (CABG), Restenose, peripherer Gefäßkrankheit, Schlaganfall, Bauchaortenaneurysma, intrakraniellem Aneurysma, Schlaganfall bei Atherosklerose großer Arterien, kardiogenem Schlaganfall, einem Myokardinfarkt im frühen Ansatz, Herzversagen, Lungenembolie, akutem Koronarsyndrom (ACS), Angina, Herzhypertrophie, Arteriosklerose, Myokarditis, Pankarditis, Endokarditis, Hypertension, kongestiver Herzinsuffizienz, Atherosklerose, zerebrovaskulärer Krankheit, abnehmender Herzgesundheit, ischämischer Herzkrankheit, Perikarditis, kardiogenem Schock, Alkoholkardiomyopathie, kongenitaler Herzkrankheit, ischämischer Kardiomyopathie, hypertensiver Kardiomyopathie, Klappenkardiomyopathie, entzündlicher Kardiomyopathie, Kardiomyopathie nach systemischer Stoffwechselkrankheit, dilatierter Kardiomyopathie, hypertropher Kardiomyopathie, arrhythmogener rechtsventrikulärer Kardiomyopathie, restriktiver Kardiomyopathie, Non-Compaction-Kardiomyopathie, Herzklappenkrankheit, hypertensiver Herzkrankheit, Myokardischämie-Attacke, instabiler Angina, Myokardruptur, kardiogenem Schock, Embolie, tiefer Venenthrombose, Arrhythmie, arrhythmogener rechtsventrikulärer Kardiomyopathie, diabetischer Kardiomyopathie, Mitralklappeninsuffizienz, Mitralklappenprolaps, peripherer Gefäßkrankheit, Arterienerkrankung, Erkrankung der Herzschlagader, tiefer Venenthrombose, Venenerkrankungen, zerebrovaskulärer Krankheit, arteriellem Aneurysma, linksventrikulärer Hypertrophie, hypertensiver Nierenerkrankung, hypertensiver Netzhauterkrankung, Vaskulitis, Erkrankung des linken Hauptbronchus, arterieller Gefäßkrankheit, venöser Gefäßkrankheit, Thrombose der Mikrozirkulation, einer transitorischen zerebrovaskulären Episode, Gliedmaßenischämie, Aneurysma, Thrombose, oberflächlicher Venenthrombose, und tiefer Venenthrombose ausgewählt wird.

11. Verfahren nach einem der Ansprüche 1 bis 4, ferner umfassend ein Extrahieren von Zellinhalten aus den phagozytischen Zellen und den nicht-phagozytischen Zelle vor a); wobei die Zellinhalte der phagozytischen Zellen gegebenenfalls lebensfähige erkrankte Zellen, tote erkrankte Zelle, apoptotische erkrankte Zellen, zirkulierende Tumorzellen, infektiöse Agenzien, fetale Zellen, Trophoblasten oder Fragmente davon umfassen.

12. Verfahren nach einem der Ansprüche 1 bis 5,
wobei die phagozytischen Zellen professionelle phagozytische Zellen, nicht-professionelle phagozytische Zellen oder Mischungen davon sind, oder wobei die nicht-phagozytischen Zellen T-Zellen, B-Zellen, Null-Zellen, Basophile oder Mischungen davon sind;
wobei die professionellen phagozytischen Zellen gegebenenfalls Neutrophile, Makrophagen, Monozyten, dendritische Zellen, Schaumzellen, Mastzellen, Eosinophile oder Mischungen davon sind;
wobei die nicht-professionellen phagozytischen Zellen gegebenenfalls Epithelzellen, Endothelzellen, Fibroblasten, mesenchymale Zellen oder Mischungen davon sind; und
wobei die phagozytischen Zellen oder die nicht-phagozytischen Zellen gegebenenfalls aus einer Körperflüssigkeitsprobe, Geweben oder Zellen des Subjekts isoliert werden; wobei es sich bei der Körperflüssigkeitsprobe gegebenenfalls um Blut, Harn, Stuhl, Speichel, Lymphflüssigkeit, Zerebrospinalflüssigkeit, Synovialflüssigkeit, zystische Flüssigkeit, Aszites, Pleuraerguss, Flüssigkeit, die von einer schwangeren Frau im ersten Trimester erhalten wird, Flüssigkeit, die von einer schwangeren Frau im zweiten Trimester erhalten wird, Flüssigkeit, die von einer schwangeren Frau im dritten Trimester erhalten wird, mütterliches Blut, Fruchtwasser, Chorionzottenprobe, mütterlichen Harn, mütterlichen Speichel, Plazentaprobe, fetales Blut, Lavage und zervikale Scheidenflüssigkeit, interstitielle Flüssigkeit oder Augenflüssigkeit handelt.

## Revendications

1. Procédé pour identifier un ou plusieurs marqueurs d'une maladie ou d'un état cardiovasculaire, comprenant :
a) la préparation d'un premier profil d'analytes par détection desdits analytes à partir de cellules phagocytaires d'un sujet, dans lequel le sujet a ladite maladie ou ledit état ;
la préparation d'un deuxième profil desdits analytes par détection desdits analytes à partir de cellules non phagocytaires du sujet ;
l'identification d'un premier jeu de différences entre les premier et deuxième profils, dans lequel le premier jeu de différences est spécifique du premier profil par rapport au deuxième profil ;
b) la préparation d'un troisième profil desdits analytes par détection desdits analytes à partir de cellules phagocytaires d'un sujet témoin, dans lequel le sujet témoin n'a pas ladite maladie ou ledit état ;
la préparation d'un quatrième profil desdits analytes par détection desdits analytes à partir de cellules non phagocytaires du sujet témoin ;
l'identification d'un deuxième jeu de différences entre les troisième et quatrième profils, dans lequel le deuxième jeu de différences est spécifique du troisième profil par rapport au quatrième profil ;
c) l'identification d'un ou plusieurs analytes spécifiques du premier jeu de différences par rapport au deuxième jeu de différences, les analytes identifiés étant des marqueurs de ladite maladie ou dudit état ;
d) l'obtention d'un cinquième profil desdits analytes à partir de cellules ou de tissus affectés par ladite maladie ou ledit état chez le sujet ayant ladite maladie ou ledit état ;
l'obtention d'un sixième profil desdits analytes à partir de cellules ou de tissus non affectés par ladite maladie ou ledit état chez le sujet ayant ladite maladie ou ledit état ;
l'identification d'un troisième jeu de différences entre les cinquième et sixième profils, dans lequel le troisième jeu de différences est spécifique du cinquième profil par rapport au sixième profil ; et
e) l'identification d'au moins un du ou des marqueurs de c) présents dans le troisième jeu de différences.

2. Procédé pour identifier un ou plusieurs marqueurs d'une maladie ou d'un état cardiovasculaire, comprenant :
a) la préparation d'un premier profil d'analytes par détection desdits analytes à partir de cellules phagocytaires d'un sujet, dans lequel le sujet a ladite maladie ou ledit état ;
la préparation d'un deuxième profil desdits analytes par détection desdits analytes à partir de cellules phagocytaires d'un sujet témoin, dans lequel le sujet témoin n'a pas ladite maladie ou ledit état ;
l'identification d'un premier jeu de différences entre les premier et deuxième profils, dans lequel le premier jeu de différences est spécifique du premier profil par rapport au deuxième profil ;
b) la préparation d'un troisième profil desdits analytes par détection desdits analytes à partir de cellules non phagocytaires du sujet ;
la préparation d'un quatrième profil desdits analytes par détection desdits analytes à partir de cellules non phagocytaires du sujet témoin ;
l'identification d'un deuxième jeu de différences entre les troisième et quatrième profils, dans lequel le deuxième jeu de différences est spécifique du troisième profil par rapport au quatrième profil ;
c) l'identification d'un ou plusieurs analytes spécifiques du premier jeu de différences par rapport au deuxième jeu de différences, les analytes identifiés étant des marqueurs de ladite maladie ou dudit état ;
d) l'obtention d'un cinquième profil desdits analytes à partir de cellules ou de tissus affectés par ladite maladie ou ledit état chez le sujet ayant ladite maladie ou ledit état ;
l'obtention d'un sixième profil desdits analytes à partir de cellules ou de tissus non affectés par ladite maladie ou ledit état chez le sujet ayant ladite maladie ou ledit état ;
l'identification d'un troisième jeu de différences entre les cinquième et sixième profils, dans lequel le troisième jeu de différences est spécifique du cinquième profil par rapport au sixième profil ; et
e) l'identification d'au moins un du ou des marqueurs de c) présents dans le troisième jeu de différences.

3. Procédé pour identifier un ou plusieurs marqueurs d'une maladie ou d'un état cardiovasculaire, comprenant :
a) la préparation d'un premier profil d'analytes par détection d'analytes à partir de cellules phagocytaires d'un sujet, dans lequel le sujet a ladite maladie ou ledit état ;
l'obtention d'un deuxième profil d'analytes à partir de cellules phagocytaires d'un sujet témoin, dans lequel le sujet témoin n'a pas ladite maladie ou ledit état, par exploration de données ;
l'identification d'un premier jeu de différences entre les premier et deuxième profils, dans lequel le premier jeu de différences est spécifique du premier profil par rapport au deuxième profil ;
b) la préparation d'un troisième profil desdits analytes par détection desdits analytes à partir des cellules non phagocytaires du sujet ;
l'obtention d'un quatrième profil desdits analytes à partir des cellules non phagocytaires du sujet témoin par exploration de données ;
l'identification d'un deuxième jeu de différences entre les troisième et quatrième profils, dans lequel le deuxième jeu de différences est spécifique du troisième profil par rapport au quatrième profil ; et
c) l'identification d'un ou plusieurs analytes spécifiques du premier jeu de différences par rapport au deuxième jeu de différences, les analytes identifiés étant des marqueurs de ladite maladie ou dudit état ;
d) l'obtention d'un cinquième profil desdits analytes à partir de cellules ou de tissus affectés par ladite maladie ou ledit état par exploration de données ;
l'obtention d'un sixième profil desdits analytes à partir de cellules ou de tissus non affectés par ladite maladie ou ledit état par exploration de données ;
l'identification d'un troisième jeu de différences entre les cinquième et sixième profils, dans lequel le troisième jeu de différences est spécifique du cinquième profil par rapport au sixième profil ; et
e) l'identification d'au moins un du ou des marqueurs de c) présents dans le troisième jeu de différences.

4. Procédé pour identifier un ou plusieurs marqueurs d'une maladie ou d'un état cardiovasculaire, comprenant :
a) la préparation d'un premier profil d'analytes par détection desdits analytes à partir de cellules phagocytaires d'un sujet, dans lequel le sujet a ladite maladie ou ledit état ;
la préparation d'un deuxième profil desdits analytes par détection desdits analytes à partir des cellules non phagocytaires du sujet ;
l'identification d'un premier jeu de différences entre les premier et deuxième profils, dans lequel le premier jeu de différences est spécifique du premier profil par rapport au deuxième profil ;
b) la préparation d'un troisième profil desdits analytes par détection desdits analytes à partir des cellules ou tissus du sujet affectés par ladite maladie ou ledit état ;
la préparation d'un quatrième profil desdits analytes par détection desdits analytes à partir des cellules ou tissus du sujet non affectés par ladite maladie ou ledit état ;
l'identification d'un deuxième jeu de différences entre les troisième et quatrième profils, dans lequel le deuxième jeu de différences est spécifique du troisième profil par rapport au quatrième profil ;
c) l'identification d'un ou plusieurs analytes présents à la fois dans le premier jeu de différences et dans le deuxième jeu de différences, les analytes identifiés étant des marqueurs de ladite maladie ou dudit état ;
d) la préparation d'un cinquième profil desdits analytes par détection desdits analytes à partir de cellules phagocytaires d'un sujet témoin, dans lequel le sujet témoin n'a pas ladite maladie ou ledit état ;
l'identification d'un troisième jeu de différences entre les premier et cinquième profils, dans lequel le troisième jeu de différences est spécifique du premier profil par rapport au cinquième profil ;
e) l'identification d'au moins un du ou des marqueurs de c) présents dans le troisième jeu de différences.

5. Procédé pour identifier un ou plusieurs marqueurs d'une maladie ou d'un état cardiovasculaire, comprenant :
a) la préparation d'un premier profil d'analytes par détection desdits analytes à partir des cellules phagocytaires > 2n d'un sujet, dans lequel le sujet a ladite maladie ou ledit état ;
la préparation d'un deuxième profil desdits analytes par détection desdits analytes à partir des cellules phagocytaires =2n du sujet ;
l'identification d'un premier jeu de différences entre les premier et deuxième profils, dans lequel le premier jeu de différences est spécifique du premier profil par rapport au deuxième profil ;
b) la préparation d'un troisième profil desdits analytes par détection desdits analytes à partir des cellules phagocytaires > 2n d'un sujet témoin, dans lequel le sujet témoin n'a pas ladite maladie ou ledit état ;
la préparation d'un quatrième profil desdits analytes par détection desdits analytes à partir des cellules phagocytaires =2n du sujet témoin ;
l'identification d'un deuxième jeu de différences entre les troisième et quatrième profils, dans lequel le deuxième jeu de différences est spécifique du troisième profil par rapport au quatrième profil ; et
c) l'identification d'un ou plusieurs analytes spécifiques du premier jeu de différences par rapport au deuxième jeu de différences, les analytes identifiés étant des marqueurs de ladite maladie ou dudit état ;
d) l'obtention d'un cinquième profil desdits analytes à partir de cellules ou de tissus affectés par ladite maladie ou ledit état chez le sujet ayant ladite maladie ou ledit état ;
l'obtention d'un sixième profil desdits analytes à partir de cellules ou de tissus non affectés par ladite maladie ou ledit état chez le sujet ayant ladite maladie ou ledit état ;
l'identification d'un troisième jeu de différences entre les cinquième et sixième profils, dans lequel le troisième jeu de différences est spécifique du cinquième profil par rapport au sixième profil ; et
e) l'identification d'au moins un du ou des marqueurs de c) présents dans le troisième jeu de différences.

6. Procédé selon l'une quelconque des revendications 1 à 5, dans lequel les analytes sont des acides nucléiques, des protéines, des lipides, des hydrates de carbone, des métabolites, ou des combinaisons de ceux-ci ;
éventuellement dans lequel les acides nucléiques sont des nucléotides, des oligonucléotides, des ADN, des ARN, ou des hybrides d'ADN-ARN ;
éventuellement dans lequel les protéines sont des acides aminés, des peptides, des enzymes, des antigènes, des anticorps, des cytokines, des lipoprotéines, des glycoprotéines, ou des hormones ;
éventuellement dans lequel les lipides sont des acides gras, des graisses neutres, des phosphatides, du cholestérol, des esters de cholestérol, des triglycérides, des glycolipides, des glycérolipides, des glycérophospholipides, des sphingolipides, des lipides de stérol, des lipides de prénol, des saccharolipides, des polycétides, du glycérophospholipide à choline, du glycérophospholipide à éthanolamine, du phosphatidylinositol, du phosphatidylglycérol, de la phosphatidylsérine, du glycérophospholipide à lysocholine, du glycérophospholipide à lysoéthanolamine, de l'acide phosphatidique, de l'acide lysophosphatidique, de la sphingomyéline, du galactosylcéramide, du glucosylcéramide, des acides gras libres, des prostaglandines, du triacylglycérol, du diacylglycérol, du monoacylglycérol, de l'acyl-CoA, de l'acylcarnitine, de l'oxystérol, du céramide, de la cardiolipine, du sphingoïde base-1-phosphate, de la sphingosine, de la lysosphingomyéline, des gangliosides, du plasmalogène, du sulfatide, des lipoprotéines basse densité (LDL), des lipoprotéines très basse densité (VLDL), des lipoprotéines haute densité (HDL), des sphingoïde base-1-phosphates ou des dérivés de ceux-ci ;
éventuellement dans lequel les hydrates de carbone sont des monosaccharides, des disaccharides, des polysaccharides, des oligosaccharides, ou des dérivés de ceux-ci ; et
éventuellement dans lequel les métabolites sont des métabolites primaires, des métabolites secondaires, des métabolites organiques, des métabolites inorganiques, des prostaglandines, des acides hydroxyéicosatétrénoïques, des acides hydroxyoctadécadiénoïques, des stéroïdes, des acides biliaires, des vitamines, ou des dérivés de ceux-ci.

7. Procédé selon l'une quelconque des revendications 1 à 6, dans lequel le profil est un profil d'acides nucléiques, un profil de protéines, un profil de lipides, un profil d'hydrates de carbone, un profil de métabolites, ou une combinaison de ceux-ci ;
éventuellement dans lequel le profil d'acides nucléiques est un profil génotypiques, un profil de polymorphismes mononucléotidiques, un profil de mutations géniques, un profil de nombres de copies géniques, un profil de méthylation d'ADN, un profil d'acétylation d'ADN, un profil de dosage des chromosomes, un profil d'expression de gènes, ou une combinaison de ceux-ci ; et
éventuellement dans lequel le profil de protéines est un profil d'expression de protéines, un profil d'activation de protéines, ou une combinaison de ceux-ci ; et en outre éventuellement dans lequel le profil d'activation de protéines comprend la détermination d'un état de phosphorylation, d'un état d'ubiquitination, d'un état de myristoylation, ou d'un état de conformation du ou des marqueurs.

8. Procédé selon l'une quelconque des revendications 1 à 7, dans lequel le profil est détecté par un dosage qualitatif, un dosage quantitatif, ou une combinaison de ceux-ci ;
éventuellement dans lequel le dosage quantitatif utilise un séquençage, un séquençage direct, un séquençage aléatoire globale, un séquençage par terminaison de chaîne au didésoxy de Sanger, un séquençage de génome complet, un séquençage par hybridation, un pyroséquençage, une électrophorèse capillaire, une électrophorèse sur gel, un séquençage en duplex, un séquençage cyclique, un séquençage par extension d'une seule base, un séquençage en phase solide, un séquençage à haut débit, un séquençage par signature massivement parallèle, une PCR en émulsion, un séquençage par terminateur colorant réversible, un séquençage par paires appariées, un séquençage à court terme, un séquençage par exonucléase, un séquençage par ligature, un séquençage par lecture courte, un séquençage d'une seule molécule, un séquençage par synthèse, un séquençage en temps réel, un séquençage par terminateur inverse, un séquençage par nanopore, un séquençage 454, un séquençage au moyen d'un analyseur de génome Solexa, un séquençage SOLiD®, un séquençage MS-PET, une spectrométrie de masse, une spectrométrie de masse par désorption laser assistée par une matrice / ionisation - temps de vol (MALDI-TOF), une spectrométrie de masse par ionisation de pulvérisation d'électrons (ESI), une spectrométrie de masse par désorption laser amplifiée en surface / ionisation - temps de vol (SELDI-TOF), une spectrométrie de masse quadripolaire - temps de vol (Q-TOF), une spectrométrie de masse par photo-ionisation sous la pression atmosphérique (APPI-MS), une spectrométrie de masse par transformée de Fourier (FTMS), une spectrométrie de masse par désorption laser assistée par une matrice / ionisation - transformée de Fourier - résonance cyclotronique ionique (MALDI-FT-ICR), une spectrométrie de masse à ions secondaires (SIMS), une analyse d'amplification en chaîne par polymérase (PCR), une PCR quantitative, une PCR en temps réel, un test de fluorescence, un test colorimétrique, un test chimioluminescent, ou une combinaison de ceux-ci.

9. Procédé selon l'une quelconque des revendications 1 à 8, dans lequel le profil est un profil d'acides nucléiques détectés par une analyse d'amplification en chaîne par polymérase (PCR), une analyse de séquençage, une analyse électrophorétique, une analyse de polymorphisme de longueur de fragment de restriction (RFLP), une analyse par transfert Northern, une analyse PCR à transcriptase inverse (RT-PCR), une PCR quantitative, une RT-PCR quantitative, une analyse d'hybridation d'oligonucléotide à spécificité d'allèle, une hybridation génomique comparative, un test de mobilité d'hétéroduplex (HMA), un polymorphisme de conformation de simple brin (SSCP), une électrophorèse sur gel à gradient de dénaturation (DGGE), une analyse de mésappariement d'ARNase, une spectrométrie de masse, une spectrométrie de masse par désorption laser assistée par une matrice / ionisation - temps de vol (MALDI-TOF), une spectrométrie de masse par ionisation de pulvérisation d'électrons (ESI), une spectrométrie de masse par désorption laser amplifiée en surface / ionisation - temps de vol (SELDI-TOF), une spectrométrie de masse quadripolaire - temps de vol (Q TOF), une spectrométrie de masse par photo-ionisation sous la pression atmosphérique (APPI-MS), une spectrométrie de masse par transformée de Fourier (FTMS), une spectrométrie de masse par désorption laser assistée par une matrice / ionisation - transformée de Fourier - résonance cyclotronique ionique (MALDI-FT-ICR), une spectrométrie de masse à ions secondaires (SIMS), une analyse par transfert Southern, une hybridation *in situ,* une hybridation *in situ* en fluorescence (FISH), une hybridation *in situ* chromogénique (CISH), une immunohistochimie (IHC), une puce à ADN, une hybridation génomique comparative, un caryotypage, une amplification par sonde dépendante d'une ligature multiplex (MLPA), une PCR multiplex quantitative de fragments fluorescents courts (QMPSF), une microscopie, un test PCR spécifique d'une méthylation (MSP), un test PCR à médiation par un enrichissement en minuscules fragments HpaII par ligature (HELP), des tests par marquage à l'acétate radioactif, un test d'acétylation d'ADN colorimétrique, une immunoprécipitation de chromatine combinée à un test sur puce à ADN (ChIP sur puce), un balayage génomique à points de repère de restriction, une immunoprécipitation d'ADN méthylé (MeDIP), un test de lumière de décomposition moléculaire pour l'activité d'ADN adénine méthyltransférase, une séparation chromatographique, une analyse d'enzyme de restriction sensible à une méthylation, une résonance plasmonique de surface, une conversion entraînée par le bisulfite de cytosine non méthylée en uracile, une analyse PCR par liaison méthyle, ou une combinaison de ceux-ci ;
éventuellement dans lequel l'analyse de séquençage est choisie dans le groupe constitué par un séquençage direct, un séquençage aléatoire globale, un séquençage par terminaison de chaîne au didésoxy de Sanger, un séquençage de génome complet, un séquençage par hybridation, un pyroséquençage, une électrophorèse capillaire, une électrophorèse sur gel, un séquençage en duplex, un séquençage cyclique, un séquençage par extension d'une seule base, un séquençage en phase solide, un séquençage à haut débit, un séquençage par signature massivement parallèle, une PCR en émulsion, un séquençage par terminateur colorant réversible, un séquençage par paires appariées, un séquençage à court terme, un séquençage par exonucléase, un séquençage par ligature, un séquençage par lecture courte, un séquençage d'une seule molécule, un séquençage par synthèse, un séquençage en temps réel, un séquençage par terminateur inverse, un séquençage par nanopore, un séquençage 454, un séquençage au moyen d'un analyseur de génome Solexa, un séquençage SOLiD®, un séquençage MS-PET, une spectrométrie de masse, et une combinaison de ceux-ci ;
ou dans lequel le profil est un profil de protéines détecté par un dosage immunohistochimique, un test immuno-enzymatique (ELISA), une chromatographie, une chromatographie en phase liquide, une chromatographie d'exclusion stérique, une chromatographie en phase liquide à haute performance (HPLC), une chromatographie gazeuse, une spectrométrie de masse, une spectrométrie de masse en tandem, une spectrométrie de masse par désorption laser assistée par une matrice / ionisation - temps de vol (MALDI-TOF), une spectrométrie de masse par ionisation de pulvérisation d'électrons (ESI), une spectrométrie de masse par désorption laser amplifiée en surface / ionisation - temps de vol (SELDI-TOF), une spectrométrie de masse quadripolaire - temps de vol (Q TOF), une spectrométrie de masse par photo-ionisation sous la pression atmosphérique (APPI-MS), une spectrométrie de masse par transformée de Fourier (FTMS), une spectrométrie de masse par désorption laser assistée par une matrice / ionisation - transformée de Fourier - résonance cyclotronique ionique (MALDI-FT-ICR), une spectrométrie de masse à ions secondaires (SIMS), des radioimmunodosages, une résonance plasmonique de surface, des tests basés sur des puces microfluidiques, un test par transfert Western, ou une combinaison de ceux-ci ;
ou dans lequel le profil est un profil de lipides détecté par une chromatographie, une chromatographie en phase liquide, une chromatographie d'exclusion stérique, une chromatographie en phase liquide à haute performance (HPLC), une chromatographie gazeuse, une spectrométrie de masse, une spectrométrie de masse par désorption laser assistée par une matrice / ionisation - temps de vol (MALDI-TOF), une spectrométrie de masse en tandem, une spectrométrie de masse par ionisation de pulvérisation d'électrons (ESI), une spectrométrie de masse par désorption laser amplifiée en surface / ionisation - temps de vol (SELDI-TOF), une spectrométrie de masse quadripolaire - temps de vol (Q-TOF), une spectrométrie de masse par photo-ionisation sous la pression atmosphérique (APPI-MS), une spectrométrie de masse par transformée de Fourier (FTMS), une spectrométrie de masse par désorption laser assistée par une matrice / ionisation - transformée de Fourier - résonance cyclotronique ionique (MALDI-FT-ICR), une spectrométrie de masse à ions secondaires (SIMS), des radioimmunodosages, des tests basés sur des puces microfluidiques, une détection de fluorescence, une détection de chimioluminescence, ou une combinaison de ceux-ci ;
ou dans lequel le profil est un profil d'hydrates de carbone détecté par une chromatographie, une chromatographie en phase liquide, une chromatographie d'exclusion stérique, une chromatographie par échange d'anions haute performance avec détection ampérométrique pulsée (HPAEC-PAD), une chromatographie en phase liquide, une chromatographie gazeuse, un dosage de fluorescence, une spectrométrie de masse, une spectrométrie de masse en tandem, une spectrométrie de masse par désorption laser assistée par une matrice / ionisation - temps de vol (MALDI-TOF), une spectrométrie de masse par ionisation de pulvérisation d'électrons (ESI), une spectrométrie de masse par désorption laser amplifiée en surface / ionisation - temps de vol (SELDI-TOF), une spectrométrie de masse quadripolaire - temps de vol (Q-TOF), une spectrométrie de masse par photo-ionisation sous la pression atmosphérique (APPI-MS), une spectrométrie de masse par transformée de Fourier (FTMS), une spectrométrie de masse par désorption laser assistée par une matrice / ionisation - transformée de Fourier - résonance cyclotronique ionique (MALDI-FT-ICR), une spectrométrie de masse à ions secondaires (SIMS), des radioimmunodosages, des tests basés sur des puces microfluidiques, une détection de fluorescence, une détection de chimioluminescence, ou une combinaison de ceux-ci.

10. Procédé selon l'une quelconque des revendications 1 à 9, dans lequel la maladie ou l'état cardiovasculaire est choisi dans le groupe constitué par un infarctus du myocarde, une maladie coronarienne, une angioplastie coronarienne transluminale percutanée (ACTP), une opération de pontage coronarien (CABG), une resténose, une maladie artérielle périphérique, un accident vasculaire, un anévrisme de l'aorte abdominale, un anévrisme intracrânien, un accident athérosclérotique d'une grosse artère, un accident vasculaire cardiogénique, un infarctus du myocarde à début précoce, une insuffisance cardiaque, une embolie pulmonaire, un syndrome coronarien aigu (ACS), un angor, une hypertrophie cardiaque, une artériosclérose, une myocardite, une pancardite, une endocardite, une hypertension, une insuffisance cardiaque congestive, une athérosclérose, une maladie cérébrovasculaire, un déclin de l'état de santé cardiaque, une maladie cardiaque ischémique, une péricardite, un choc cardiogénique, une cardiomyopathie alcoolique, une maladie cardiaque congénitale, une cardiomyopathie ischémique, une cardiomyopathie d'hypertension, une cardiomyopathie valvulaire, une cardiomyopathie inflammatoire, une cardiomyopathie secondaire à une maladie métabolique systémique, une cardiomyopathie dilatée, une cardiomyopathie hypertrophique, une cardiomyopathie ventriculaire droite arythmogénique, une cardiomyopathie restrictive, une cardiomyopathie sans compactage, une maladie cardiaque valvulaire, une maladie cardiaque d'hypertension, une attaque ischémique myocardique, un angor instable, une rupture myocardique, un choc cardiogénique, une embolie, une thrombose veineuse profonde, une arythmie, une cardiomyopathie ventriculaire droite arythmogénique, une cardiomyopathie diabétique, une régurgitation mitrale, un affaissement de la valve mitrale, une maladie vasculaire périphérique, une maladie artérielle, une maladie carotidienne, une thrombose veineuse profonde, les maladies veineuses, une maladie cérébrovasculaire, un anévrisme artériel, une hypertrophie ventriculaire gauche, une maladie rénale d'hypertension, une maladie rétinienne d'hypertension, une angéite, une maladie du tronc coronaire gauche, une maladie vasculaire artérielle, une maladie vasculaire veineuse, une thrombose de la microcirculation, un accident cérébrovasculaire transitoire, une ischémie d'un membre, un anévrisme, une thrombose, une thrombose veineuse superficielle, et une thrombose veineuse profonde.

11. Procédé selon l'une quelconque des revendications 1 à 4, comprenant en outre l'extraction de contenus cellulaires à partir des cellules phagocytaires et des cellules non phagocytaires avant a) ; éventuellement dans lequel les contenus cellulaires des cellules phagocytaires comprennent des cellules malades viables, des cellules malades mortes, des cellules malades apoptotiques, des cellules tumorales en circulation, des agents infectieux, des cellules foetales, des trophoblastes, ou des fragments de ceux-ci.

12. Procédé selon l'une quelconque des revendications 1 à 5, dans lequel les cellules phagocytaires sont des cellules phagocytaires professionnelles, des cellules phagocytaires non professionnelles, ou des mélanges de celles-ci, ou dans lequel les cellules non phagocytaires sont des cellules T, des cellules B, des cellules nulles, des basophiles, ou des mélanges de ceux-ci ;
éventuellement dans lequel les cellules phagocytaires professionnelles sont des neutrophiles, des macrophages, des monocytes, des cellules dendritiques, des cellules spumeuses, des mastocytes, des éosinophiles, ou des mélanges de ceux-ci ;
éventuellement dans lequel les cellules phagocytaires non professionnelles sont des cellules épithéliales, des cellules endothéliales, des fibroblastes, des cellules mésenchymateuses, ou des mélanges de ceux-ci ; et
éventuellement dans lequel les cellules phagocytaires ou les cellules non phagocytaires sont isolées à partir d'un échantillon de fluide corporel, de tissus, ou de cellules du sujet ; éventuellement dans lequel l'échantillon de fluide corporel est du sang, de l'urine, des selles, de la salive, de la lymphe, du liquide céphalo-rachidien, du fluide synovial, du fluide cystique, du fluide ascitique, une effusion pleurale, un fluide obtenu à partir d'une femme enceinte au premier trimestre, un fluide obtenu à partir d'une femme enceinte au deuxième trimestre, un fluide obtenu à partir d'une femme enceinte au troisième trimestre, du sang maternel, du liquide amniotique, un échantillon de villosité chorionique, de l'urine maternelle, de la salive maternelle, un échantillon de placenta, du sang fatal, du fluide de lavage et vaginal cervical, du fluide interstitiel, ou du fluide oculaire.
